# EUROPÄISCHE PATENTSCHRIFT

(11) **EP 1 720 546 B1**
(45) Veröffentlichungstag und Bekanntmachung des Hinweises auf die Patenterteilung: **01.09.2010**
(21) Anmeldenummer: 05707250.6
(22) Anmeldetag: 08.02.2005
(51) Int. Cl.: A61K 31/4196, A61P 11/06, C07D 249/08, C07D 405/04

(54) **NEUE LANGWIRKSAME BETA-2-AGONISTEN, UND DEREN VERWENDUNG ALS ARZNEIMITTEL**
NOVEL, SUSTAINED-ACTION BETA-2-AGONISTS AND THEIR USE AS MEDICAMENTS
NOUVEAUX BETA-2-AGONISTES A ACTION LONGUE DUREE ET LEUR UTILISATION COMME MEDICAMENTS

(30) Priorität: 14.02.2004 EP 04003354
(43) Veröffentlichungstag der Anmeldung: 15.11.2006
(73) Patentinhaber: Boehringer Ingelheim International GmbH, 55216 Ingelheim am Rhein (DE); Boehringer Ingelheim Pharma GmbH & Co.KG, 55216 Ingelheim am Rhein (DE)
(72) Erfinder: BOUYSSOU, Thierry, 88447 Birkenhard (DE); HOENKE, Christoph, 55218 Ingelheim (DE); KONETZKI, Ingo, 88447 Warthausen (DE); MACK, Jürgen, 88400 Biberach (DE); SCHNAPP, Andreas, 88400 Biberach (DE)
(74) Vertreter: Hammann, Heinz
(86) Internationale Anmeldenummer: PCT/EP2005/001232
(87) Internationale Veröffentlichungsnummer: WO 2005/077361

(56) Entgegenhaltungen:
- EP-A- 0 008 653
- WO-A-01/83462
- WO-A-2004/039784

## Beschreibung

Die vorliegende Erfindung betrifft die Verwendung von Verbindungen der Formel **1** 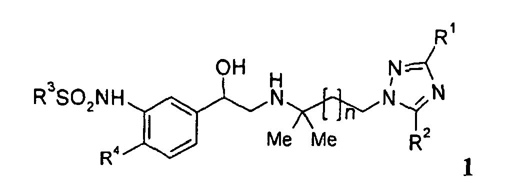 worin die Gruppen R¹, R², R³, R⁴ und n die in den Ansprüchen und in der Beschreibung genannten Bedeutungen haben können, zur Herstellung eines Arzneimittels zur Behandlung von entzündlichen und obstruktiven Atemwegserkrankungen, sowie neue Verbindungen der Formel **1** als solche.

### HINTERGRUND DER ERFINDUNG

Betamimetika (β-adrenerge Substanzen) sind aus dem Stand der Technik bekannt.

Zur medikamentösen Therapie von Erkrankungen ist es häufig wünschenswert, Arzneimittel mit einer längeren Wirkungsdauer bereitzustellen. Hierdurch kann in der Regel gewährleistet werden, dass die zur Erzielung des therapeutischen Effekts erforderliche Konzentration des Wirkstoffs im Organismus über einen längeren Zeitraum gegeben ist, ohne eine allzu häufige, wiederholte Gabe des Arzneimittels durchführen zu müssen. Die Applikation eines Wirkstoffs in längeren zeitlichen Abständen trägt im Übrigen in hohem Maße zum Wohlbefinden des Patienten bei.

Besonders wünschenswert ist die Bereitstellung eines Arzneimittels, welches therapeutisch sinnvoll durch einmalige Applikation pro Tag (Einmalgabe) eingesetzt werden kann. Eine einmal pro Tag erfolgende Anwendung hat den Vorteil, dass der Patient sich relativ schnell an die regelmäßige Einnahme des Medikaments zu bestimmten Tageszeiten gewöhnen kann.

Es ist daher Aufgabe der vorliegenden Erfindung, Betamimetika bereitzustellen, die durch eine längere Wirkdauer gekennzeichnet sind und somit zur Herstellung von Arzneimitteln mit längerer Wirksamkeit Verwendung finden können. Es ist insbesondere Aufgabe der vorliegenden Erfindung, Betamimetika bereitzustellen, die aufgrund ihrer langen Wirksamkeit zur Herstellung eines einmal täglich applizierbaren Arzneimittels eingesetzt werden können. Ein weiteres Ziel der vorliegenden Erfindung ist die Bereitstellung von neuen Betamimetika, die aufgrund ihrer langen Wirksamkeit zur Herstellung eines einrnal täglich applizierbaren Arzneimittels zur Behandlung von entzündlichen oder obstruktiven Atemwegserkrankungen verwendet werden können. Neben den vorstehend genannten Aufgaben ist es ferner Ziel der vorliegenden Erfindung, solche Betamimetika bereitzustellen, die nicht nur außerordentlich potent, sondern ferner durch ein hohes Maß an Selektivität gegenüber dem β2-Adrenozeptor gekennzeichnet sind.

### BESCHREIBUNG DER ERFINDUNG

Überraschenderweise wurde gefunden, dass die vorstehend genannten Aufgaben durch Verbindungen der Formel **1** gelöst werden. Dementsprechend betrifft die vorliegende Erfindung die Verwendung von Verbindungen der Formel **1** 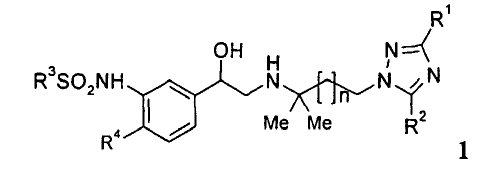 worin
- R¹: Wasserstoff, -C₁₋₆-alkyl, -C₁₋₆-haloalkyl, -OH, -O-C₁₋₆-alkyl, Halogen oder ein Rest ausgewählt aus der Gruppe Aryl oder Heterocyclus, wobei Aryl oder Heterocyclus, wenn möglich jeweils optional substituiert mit 1, 2, 3, 4 oder 5 gleichen oder unterschiedlichen Resten R⁵ sind;
- R²: Methyl;
- R³: -C₁₋₆-alkyl; bevorzugt Methyl;
- R⁴: -OH;
- R⁵: Halogen, -CN, -NO₂, -C₁₋₆-alkyl, -C₃₋₆-cycloalkyl, -C₁₋₆-haloalkyl, -COR⁶, -COOR⁶, -CONR⁶R⁷, -OR⁶, -NR⁶R⁷, -NR⁶COR⁷, -NR⁶SO₂R⁷, -SR⁶, -SOR⁶, -SO₂R⁶ oder -SO₂NR⁶R⁷, oder zwei R⁵ miteinander verbunden eine Gruppe ausgewählt aus -C₂₋₆-alkylen, -C₂₋₆-alkenylen und -O-C₁₋₆-alkylen-O-;
- R⁶ und R⁷: Wasserstoff, -C₁₋₆-alkyl, -C₃₋₆-cycloalkyl;
- n: 0, 1, 2 oder 3; bevorzugt 1;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, zur Herstellung eines Arzneimittels zur Behandlung von entzündlichen und obstruktiven Atemwegserkrankungen

Besonders bevorzugt ist die oben genannte Verwendung von Verbindungen der Formel **1** worin R², R³, R⁴ und n die oben genannte Bedeutung haben und
- R¹: Wasserstoff, -C₁₋₆-alkyl, -C₁₋₆-haloalkyl, -OH, -O-C₁₋₆-alkyl, Halogen oder Aryl, wenn möglich optional substituiert mit 1, 2, 3, 4 oder 5 gleichen oder unterschiedlichen Resten R⁵;
- R⁵: Halogen, -CN, -NO₂, -C₁₋₆-alkyl, -C₃₋₆-cycloalkyl, -C₁₋₆-haloalkyl, -COR⁶, -COOR⁶, -CONR⁶R⁷, -OR⁶, -NR⁶R⁷, -NR⁶COR⁷, -NR⁶SO₂R⁷, -SR⁶, -SOR⁶, -SO₂R⁶ oder -SO₂NR⁶R⁷, oder zwei R⁵ miteinander verbunden eine Gruppe ausgewählt aus -C₂₋₆-alkylen, -C₂₋₆-alkenylen und -O-C₁₋₆-alkylen-O-;
- R⁶ und R⁷: Wasserstoff, -C₁₋₆-alkyl, -C₃₋₆-cycloalkyl;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Besonders bevorzugt ist die oben genannte Verwendung von Verbindungen der Formel **1** worin
- R¹: Wasserstoff, -C₁₋₆-alkyl, -O-C₁₋₆-alkyl, Aryl, wenn möglich optional substituiert mit 1, 2, 3, 4 oder 5 gleichen oder unterschiedlichen Resten R⁵;
- R²: bevorzugt Methyl;
- R³: Methyl;
- R⁴: -OH;
- R⁵: Halogen, -CN, -NO₂, -C₁₋₆-alkyl, -C₃₋₆-cycloalkyl, -C₁₋₆-haloalkyl, -COR⁶, -COOR⁶, -CONR⁶R⁷, -OR⁶, -NR⁶R⁷, -NR⁶COR⁷, -NR⁶SO₂R⁷, -SR⁶, -SOR⁶, -SO₂R⁶ oder -SO₂NR⁶R⁷, oder zwei R⁵ miteinander verbunden eine Gruppe ausgewählt aus -C₂₋₆-alkylen, -C₂₋₆-alkenylen und -O-C₁₋₆-alkylen-O-;
- R⁶ und R⁷: Wasserstoff, -C₁₋₆-alkyl, -C₃₋₆-cycloalkyl;
- n: 0, 1, 2 oder 3; bevorzugt 1;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Besonders bevorzugt ist die oben genannte Verwendung von Verbindungen der Formel **1** worin
- R¹: Wasserstoff, Aryl, wenn möglich optional substituiert mit 1, 2, 3, 4 oder 5 gleichen oder unterschiedlichen Resten R⁵;
- R²: Methyl;
- R³: Methyl;
- R⁴: -OH;
- R⁵: Halogen, -CN, -NO₂, -C₁₋₆-alkyl, -C₃₋₆-cycloalkyl, -C₁₋₆-haloalkyl, -COR⁶, -COOR⁶, -CONR⁶R⁷, -OR⁶, -NR⁶R⁷, -NR⁶COR⁷, -NR⁶SO₂R⁷, -SR⁶, -SOR⁶, -SO₂R⁶ oder -SO₂NR⁶R⁷, oder zwei R⁵ miteinander verbunden eine Gruppe ausgewählt aus -C₂₋₆-alkylen, -C₂₋₆-alkenylen und -O-C₁₋₆-alkylen-O-;
- R⁶ und R⁷: Wasserstoff, -C₁₋₆-alkyl, -C₃₋₆-cycloalkyl;
- n: 0, 1, 2 oder 3; bevorzugt 1;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Besonders bevorzugt ist die oben genannte Verwendung von Verbindungen der Formel **1** worin
- R¹: Wasserstoff, Phenyl, optional substituiert mit 1, 2, 3, 4 oder 5 gleichen oder unterschiedlichen Resten R⁵;
- R²: Methyl;
- R³: Methyl;
- R⁴: OH;
- R⁵: Halogen, -CN, -NO₂, -C₁₋₆-alkyl, -C₃₋₆-cycloalkyl, -C₁₋₆-haloalkyl, -COR⁶, -COOR⁶, -CONR⁶R⁷, -OR⁶, -NR⁶R⁷, -NR⁶COR⁷, -NR⁶SO₂R⁷, -SR⁶, -SOR⁶, -SO₂R⁶ oder -SO₂NR⁶R⁷, oder zwei R⁵ miteinander verbunden eine Gruppe ausgewählt aus -C₂₋₆-alkylen, -C₂₋₆-alkenylen und -O-C₁₋₆-alkylen-O-;
- R⁶ und R⁷: Wasserstoff, -C₁₋₆-alkyl, -C₃₋₆-cycloalkyl;
- n: 1;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Besonders bevorzugt ist die vorstehend genannte Verwendung der Verbindungen der allgemeinen Formel **1** zur Herstellung eines Arzneimittels zur Behandlung von Atemwegserkrankungen, die ausgewählt sind aus der Gruppe bestehend aus Obstruktive Lungenerkrankungen unterschiedlicher Genese, Lungenemphyseme unterschiedlicher Genese, Restriktive Lungenerkrankungen, Interstitielle Lungenerkrankungen, Zystische Fibrose, Bronchitiden unterschiedlicher Genese, Bronchiektasen, ARDS (adult respiratory distress syndrom) und alle Formen des Lungenödems.

Bevorzugt ist vorstehend genannte Verwendung zur Herstellung eines Arzneimittels zur Behandlung von obstruktiven Lungenerkrankungen, die ausgewählt sind aus der Gruppe bestehend aus Asthma Bronchiale, pädiatrisches Asthma, schweres Asthma, akuter Asthma-Anfall, chronische Bronchitis und COPD (chronisch obstruktive pulmonale Erkrankung), wobei die Verwendung zur Herstellung eines Arzneimittels zur Behandlung von Asthma Bronchiale (im Rahmen der vorliegenden Erfindung gegebenenfalls auch nur als Asthma bezeichnet) und COPD erfindungsgemäß besonders bevorzugt ist.

Bevorzugt ist ferner die vorstehend genannte Verwendung zur Herstellung eines Arzneimittels zur Behandlung von Lungenemphysemen die ihren Ursprung haben in COPD oder α1-Proteinase-Inhibitor-Mangel.

Bevorzugt ist ferner die vorstehend genannte Verwendung zur Herstellung eines Arzneimittels zur Behandlung von Restriktiven Lungenerkrankungen, die ausgewählt sind aus der Gruppe bestehend aus Allergische Alveolitis, durch berufliche Noxen ausgelöste restriktive Lungenerkrankungen wie Asbestose oder Silikose und Restriktion aufgrund von Lungentumoren, wie beispielsweise Lymphangiosis carcinomatosa, brochoalveoläres Karzinom und Lymphome.

Bevorzugt ist ferner die vorstehend genannte Verwendung zur Herstellung eines Arzneimittels zur Behandlung von Interstitiellen Lungenerkrankungen, die ausgewählt sind aus der Gruppe bestehend aus infektiös bedingte Pneumonien, wie beispislweise aufgrund einer Infektion mit Viren, Bakterien, Pilzen, Protozoen, Helminthen oder anderen Erregern, Pneumonitis aufgrund unterschiedlicher Genese, wie beispielsweise Aspiration und Linksherzinsuffizienz, Strahlen-induzierte Pneumonitis oder Fibrose, Kollagenosen, wie beispielsweise Lupus erythematodes, sytemische Sklerodermie oder Sarkoidose, Granulomatosen, wie beispielsweise Morbus Boeck, idiopathische interstitielle Pneumonie oder idiopathische pulmonäre Fibrose (IPF).

Bevorzugt ist ferner die vorstehend genannte Verwendung zur Herstellung eines Arzneimittels zur Behandlung von Zystischer Fibrose bzw. Mukoviszidose.

Bevorzugt ist ferner die vorstehend genannte Verwendung der erfindungsgemäßen Arzneimittelkombinationen zur Herstellung eines Arzneimittels zur Behandlung von Bronchitiden, wie beispielsweise Bronchitis aufgrund bakterieller oder viraler Infektion, Allergische Bronchitis und Toxische Bronchitis.

Bevorzugt ist ferner die vorstehend genannte Verwendung zur Herstellung eines Arzneimittels zur Behandlung von Bronchiektasen.

Bevorzugt ist ferner die vorstehend genannte Verwendung zur Herstellung eines Arzneimittels zur Behandlung von ARDS (adult respiratory distress syndrom).

Bevorzugt ist ferner die vorstehend genannte Verwendung zur Herstellung eines Arzneimittels zur Behandlung von Lungenödemen, beispielsweise toxischer Lungenödeme nach Aspiration oder Inhalation von toxischen Substanzen und Fremdstoffen.

Besonders bevorzugt betrifft die vorstehend genannte Verwendung zur Herstellung eines Arzneimittels zur Behandlung von Asthma oder COPD. Von besonderer Bedeutung ist ferner die vorstehend genannte Verwendung der erfindungsgemäßen Arzneimittelkombinationen zur Herstellung eines Arzneimittels zur einmal täglichen Behandlung von entzündlichen und obstruktiven Atemwegserkrankungen, besonders bevorzugt zur einmal täglichen Behandlung von Asthma oder COPD.

Die Erfindung betrifft ferner neue Verbindungen der Formel **1** als solche. Insbesondere betrifft die vorliegende Erfindung neue Verbindungen der Formel **1** worin R¹ und n die vorstehend genannten Bedeutungen haben können, R² Methyl oder Ethyl bedenten, und worin R³ Methyl und R⁴ OH bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Bevorzugt sind ferner Verbindungen der Formel **1** worin R¹ die vorstehend genannten Bedeutungen haben kann und worin
- R²: Methyl oder Ethyl;
- R³: Methyl;
- R⁴: OH und
- n: 1
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Erfindungsgemäß bevorzugt sind Verbindungen der Formel **1** worin
- R¹: Wasserstoff, Phenyl, optional substituiert mit 1, 2 oder 3 gleichen oder unterschiedlichen Resten R⁵;
- R²: Methyl oder Ethyl, bevorzugt Methyl;
- R³: Methyl;
- R⁴: OH;
- R⁵: Halogen, -C₁₋₆-alkyl, -C₃₋₆-cycloalkyl, -C₁₋₆-haloalkyl, -COOR⁶, -CONR⁶R⁷, -OR⁶, -NR⁶R⁷, -NR⁶COR⁷, -NR⁶SO₂R⁷ oder zwei R⁵ miteinander verbunden eine Gruppe ausgewählt aus -C₂₋₆-alkylen, -C₂₋₆-alkenylen und -O-C₁₋₆-alkylen-O-;
- R⁶ und R⁷: Wasserstoff, -C₁₋₆-alkyl, -C₃₋₆-cycloalkyl;
- n: 1;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Bevorzugt sind ferner Verbindungen der Formel **1** worin
- R¹: Wasserstoff, Phenyl, optional substituiert mit 1, 2 oder 3 gleichen oder unterschiedlichen Resten R⁵;
- R²: Ethyl oder Methyl; bevorzugt Methyl;
- R³: Methyl;
- R⁴: OH;
- R⁵: Halogen, -C₁₋₆-alkyl, -C₁₋₆-haloalkyl, -COOR⁶, -CONR⁶R⁷, -OR⁶, -NR⁶R⁷ oder zwei R⁵ miteinander verbunden bedeuten -O-C₁₋₆-alkylen-O-;
- R⁶ und R⁷: Wasserstoff, -C₁₋₆-alkyl;
- n: 1;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Besonders bevorzugt sind Verbindungen der Formel **1** worin
- R¹: Phenyl, optional substituiert mit 1, 2 oder 3 gleichen oder unterschiedlichen Resten R⁵_{;}
- R²: Methyl, Ethyl; bevorzugt Methyl;
- R³: Methyl;
- R⁴: OH;
- R⁵: Chlor, Brom, Fluor, Methyl, Ethyl, -CF₃, -COOH, -COOMe, -OH, -OMe;
- n: 1
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Besonders bevorzugte Verbindungen der Formel **1** sind ausgewählt aus der Gruppe bestehend aus:
- N-[5-(2-{3-[5-Ethyl-3-(4-methoxy-phenyl)-[1,2,4]triazol-1-yl]-1,1-dimethyl-propylamino}-1-hydroxy-ethyl)-2-hydroxy-phenyl]-methansulfonamid,
- N-[5-(2-{1,1-Dimethyl-3-[5-methyl-3-(4-trifluoromethyl-phenyl)-[1,2,4]triazol-1-yl]-propylamino}-1-hydroxy-ethyl)-2-hydroxy-phenyl]-methansulfonamid,
- N-(5-{2-[1,1-Dimethyl-3-(5-methyl-3-p-tolyl-[1,2,4]triazol-1-yl)-propylamino]-1-hydroxy-ethyl}-2-hydroxy-phenyl)-methansulfonamid,
- N-[5-(2-{3-[3-(4-Fluoro-phenyl)-5-methyl-[1,2,4]triazol-1-yl)-1,1-dimethyl-propylamino)-1-hydroxy-ethyl)-2-hydroxy-phenyl]-methansulfonamid,
- 3-(1-{3-[2-Hydroxy-2-(4-hydroxy-3-methansulfonylamino-phenyl)-ethylamino]-3-methyl-butyl}-5-methyl-1H-[1,2,4]triazol-3-yl)-benzoesäuremethylester,
- N-[5-(2-{3-[3-(3,5-Difluor-phenyl)-5-methyl-[1,2,4]triazol-1-yl]-1,1-dimethyl-propylamino}-1-hydroxy-ethyl)-2-hydroxy-phenyl]-methansulfonamid,
- N-[2-Hydroxy-5-(1-hydroxy-2-{3-[3-(2-methoxy-phenyl)-5-methyl-[1,2,4]triazol-1-yl] -1,1-dimethyl-propylamino}-ethyl)phenyl]-methansulfonamid,
- N-[2-Hydroxy-5-(1-hydroxy-2-{3-[3-(4-methoxy-phenyl)-5-methyl-[1,2,4]triazol-1-yl] -1,1-dimethyl-propylamino}-ethyl)-phenyl]-methansulfonamid,
- N-(5-{2-[3-(3-Benzo[1,3]dioxol-5-yl-5-methyl-[1,2,4]triazol-1-yl)-1,1-dimethyl-propyl amino]-1-hydroxy-ethyl}-2-hydroxy-phenyl)-methansulfonamid,
- N-[2-Hydroxy-5-(1-hydroxy-2-{3-[3-(4-methoxy-phenyl)-[1,2,4]triazol-1-yl]-1,1-dimet hyl-propylamino}-ethyl)-phenyl]-methansulfonamid und
- N-{5-[2-[1,1-Dimethyl-3-[1,2,4]triazol-1-yl-propylamino)-1-hydroxy-ethyl]-2-hydroxy -phenyl}-methansulfonamid,
gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Die Verbindungen der Formel **1** können gegebenenfalls in Form der einzelnen optischen Isomeren, Mischungen der einzelnen Enantiomeren oder Racemate zur Anwendung gelangen. Besonders bevorzugt gelangen Sie in Form der enantiomerenreinen Verbindungen zur Anwendung, wobei die Verbindungen der Formel **1**, in denen das zum Phenylring benzylische, asymmetrische Kohlenstoffzentrum "-CH(OH)-" *R*-konfiguriert ist. Die besonders bevorzugten *R*-Enantiomere der Verbindungen der allgemeinen Formel **1** sind durch die allgemeine Formel ***R*-1** darstellbar, 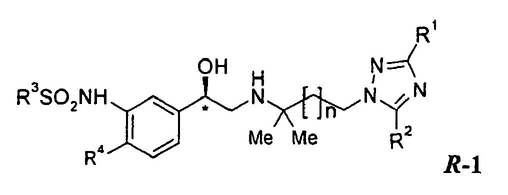 worin die Gruppen R¹, R², R³, R⁴ und n die vorstehend genannten Bedeutungen haben können.

Unter Säureadditionssalzen mit pharmakologisch unbedenklichen Säuren werden dabei beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-*p*-toluolsulfonat, bevorzugt Hydrochlorid, Hydrobromid, Hydrosulfat, Hydrophosphat, Hydrofumarat und Hydromethansulfonat verstanden.

Halogen steht im Rahmen der vorliegenden Erfindung für Fluor, Chlor, Brom oder Jod. Sofern nicht gegenteilig angegeben, gelten Fluor und Chlor als bevorzugte Halogene, wobei Fluor im Allgemeinen bevorzugt ist.

Als Alkylgruppen (Alkyl) werden, soweit nicht anders angegeben, verzweigte und unverzweigte Alkylgruppen mit 1 bis 6, bevorzugt 1 bis 4 Kohlenstoffatomen bezeichnet. Beispielsweise werden genannt: Methyl, Ethyl, Propyl oder Butyl. Zur Bezeichnung der Gruppen Methyl, Ethyl, Propyl oder auch Butyl werden gegebenenfalls auch die Abkürzungen Me, Et, Prop oder Bu verwendet. Sofern nicht anders beschrieben, umfassen die Definitionen Propyl und Butyl alle denkbaren isomeren Formen der jeweiligen Reste. So umfasst beispielsweise Propyl n-Propyl und *iso*-Propyl, Butyl umfasst *iso*-Butyl, *sec*-Butyl und *tert*-Butyl etc.

Als Alkylengruppen (Alkylen) werden, soweit nicht anders angegeben, verzweigte und unverzweigte Alkylengruppen mit 1 bis 6, bevorzugt 1 bis 4 Kohlenstoffatomen bezeichnet. Beispielsweise werden genannt: Methylen, Ethylen, Propylen oder Butylen.

Sofern nicht anders beschrieben, umfassen die Definitionen Propylen und Butylen alle denkbaren isomeren Formen der jeweiligen Reste.

Als Alkenylengruppen (Alkenylen) werden, soweit nicht anders angegeben, verzweigte und unverzweigte Alkenylengruppen mit 1 bis 6, bevorzugt 1 bis 4 Kohlenstoffatomen bezeichnet. Beispielsweise werden genannt: Ethenylen, Propenylen oder Butenylen.

Als Cycloalkylgruppen (Cycloalkyl) werden, soweit nicht anders angegeben cyclische Alkylgruppen mit 3 bis 6 bezeichnet. Beispielsweise werden genannt: Cyclopropyl, Cyclobutanyl, Cyclopentyl oder Cyclohexyl.

Als Alkyloxygruppen (O-Alkyl) werden, soweit nicht anders angegeben, verzweigte und unverzweigte Alkylgruppen mit 1 bis 6, bevorzugt 1 bis 4 Kohlenstoffatomen bezeichnet, die über ein Sauerstoffatom verknüpft sind. Beispielsweise werden genannt: Methyloxy, Ethyloxy, Propyloxy oder Butyloxy. Zur Bezeichnung der Gruppen Methyloxy, Ethyloxy, Propyloxy oder auch Butyloxy werden gegebenenfalls auch die Abkürzungen -OMe, -OEt, -OProp oder -OBu verwendet. Sofern nicht anders beschrieben, umfassen die Definitionen Propyloxy und Butyloxy alle denkbaren isomeren Formen der jeweiligen Reste. So umfasst beispielsweise Propyloxy *n*-Propyloxy und *iso*-Propyloxy, Butyloxy umfasst *iso*-Butyloxy, *sec*-Butyloxy und *tert*-Butyloxy etc. Gegebenenfalls wird im Rahmen der vorliegenden Erfindung statt der Bezeichnung Alkyloxy auch die Bezeichnung Alkoxy verwendet. Zur Bezeichnung der Gruppen Methyloxy, Ethyloxy, Propyloxy oder auch Butyloxy gelangen dementsprechend gegebenenfalls auch die Ausdrücke Methoxy, Ethoxy, Propoxy oder Butoxy zur Anwendung.

Als Halogenalkylengruppen (Haloalkyl) werden, soweit nicht anders angegeben, verzweigte und unverzweigte Alkylgruppen mit 1 bis 6 Kohlenstoffatomen bezeichnet, bei denen ein oder mehrere Wasserstoffatome durch Halogenatome, bevorzugt durch Fluor ersetzt sind. Beispielsweise werden genannt: CHF₂, CF₃, CH₂CF₃, CF₂CF₃.

Als Arylgruppen werden, soweit nicht anders angegeben, aromatische Ringsysteme mit 6 bis 10 Kohlenstoffatomen bezeichnet. Bevorzugte Arylgruppen sind Phenyl und Naphthyl, wobei Phenyl erfindungsgemäß besonders bevorzugt ist.

Als heterocyclische Gruppen (Heterocyclus) werden, soweit nicht anders angegeben, aromatische oder nicht aromatische Ringsysteme mit 2 bis 5 Kohlenstoffatomen und 1, 2 oder 3 Atomen ausgewählt aus der Gruppe O, S oder N, bevorzugt N, bezeichnet. Besonders bevorzugte Heterocyclen sind Piperidin, Piperazin, Morpholin, Pyrolidin, Pyrrol, Imidazol, Triazol, Pyridin, Pyrimidin, Thiophen, Tetrahydrofuran oder Furan.

Die Herstellung der erfindungsgemäßen Verbindungen kann in Analogie zu im Stand der Technik bereits bekannten Vorgehensweisen erfolgen. Geeignete Herstellverfahren sind beispielsweise aus der EP 43 940 oder auch aus der WO 01/83462 bekannt, auf die an dieser Stelle vollinhaltlich Bezug genommen wird.

Die nachstehend beschriebenen Synthesebeispiele dienen der weitergehenden Illustration von neuen erfindungsgemäßen Verbindungen. Sie sind allerdings nur als exemplarische Vorgehensweisen zur weitergehenden Erläuterung der Erfindung zu verstehen, ohne selbige auf den nachfolgend exemplarisch beschriebenen Gegenstand zu beschränken.

### Zwischenprodukt 1: 1,1-Dimethyl-3-(5-methyl-3-p-tolyl-[1,2,4]triazol-1-yl]-propylamin

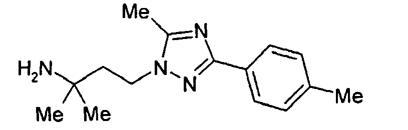

### a) 4-Methyl-benzoesäure-(1-imino-ethyl)-hydrazid

1.65 g (72 mmol) Natrium werden in 80 mL Ethanol gelöst. 8.89 g (72 mmol) Ethylacetimidat hydrochlorid in 160 mL Ethanol werden bei Raumtemperatur zugegeben und das ausfallende Natriumchlorid wird abfiltriert. Das Filtrat wird mit 6.00 g (40 mmol) 4-Methyl-benzoesäure hydrazid versetzt und über Nacht gerührt. Die Reaktionsmischung wird eingeengt und gekühlt. Der ausfallende Feststoff wird abfiltriert und mit kaltem Ethanol und Diethylether gewaschen (5.7 g weißer Feststoff). Aus dem Filtrat werden nach dem Abdestillieren der Lösungsmittel und Umkristallisation aus Ethanol weitere 1.2 g Feststoff gewonnen. Ausbeute: 6.93 g (91%); Massenspektroskopie [M+H]⁺ = 192.

### b) 5-Methyl-3-p-tolyl-[1,2,4]triazol

7.58 g (40 mmol) 4-Methyl-benzoesäure-(1-imino-ethyl)-hydrazid werden unter Rühren für 30 Minuten auf 180°C erwärmt. Nach Abkühlung wird der Feststoff in Chloroform gelöst. Der bei Kühlung ausfallende Niederschlag wird abgesaugt und aus Chloroform umkristallisiert. Ausbeute: 4.82 g (70%); Massenspektroskopie [M+H]⁺ = 174.

### c) [1,1-Dimethyl-3-(5-methyl-3-p-tolyl-[1,2,4]triazol-1-yl)-propyl]-carbaminsäure tert-butyl ester

Zu einer Lösung von 4.87 g (28 mmol) 5-Methyl-3-p-tolyl-[1,2,4]triazol in 40 mL DMPU werden bei 0°C 1.35 g (34 mmol, 60%ig) Natriumhydrid gegeben. Die Reaktionsmischung wird auf Raumtemperatur erwärmt und dann eine Stunde gerührt. 9.35 g (42 mmol) (3-Chlor-1,1-dimethyl-propyl)-carbaminsäure-tert-butylester und 1.87 g (5 mmol) Tetrabutylammoniumiodid werden zugegeben und es wird über Nacht bei Raumtemperatur und anschließend noch 2 Stunden bei 80°C gerührt. Man versetzt mit Wasser und Ethylacetat, trennt die wässrige Phase ab und extrahiert diese mit Ethylacetat. Die vereinigten organischen Phasen werden mit Wasser und Natriumchlorid-Lösung gewaschen, mit Natriumsulfat getrocknet und eingeengt. Der Rückstand wird mittels Säulenchromatographie gereinigt (Kieselgel; Petrolether/Ethylacetat = 1:1). Öl. Ausbeute: 2.97 g (30%); Massenspektroskopie [M+H]⁺ = 359.

### d) 1,1-Dimethyl-3-(5-methyl-3-p-tolyl-[1,2,4]triazol-1-yl]-propylamin

Zu eine Lösung von 2.97 g (8.3 mmol) [1,1-Dimethyl-3-(5-methyl-3-p-tolyl-[1,2,4]triazol-1-yl)-propyl]-carbaminsäure-tert-butyl ester in 80 mL Dichlormethan werden insgesamt 11 mL Trifluoressigsäure getropft und es wird über Nacht bei Raumtemperatur gerührt. Das Lösungsmittel wird abdestilliert und der Rückstand mit Diethylether versetzt und gerührt. Der ausfallende Feststoff wird abfiltriert und gewaschen. Ausbeute: 2.11 g (68%, Trifluoracetat); Massenspektroskopie [M+H]⁺ = 259.

### Zwischenprodukt 2: 3-[3-(4-Fluor-phenyl)-5-methyl-[1,2,4]triazol-1-yl]-1,1-dimethylpropylamin

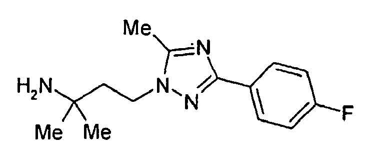

### a) 4-Fluor-benzoesäure-(1-imino-ethyl)-hydrazid

Hergestellt aus 7.2 g (58 mmol) Ethylacetimidat hydrochlorid und 5.00 g (32 mmol) 4-Fluor-benzoesäure hydrazid in Analogie zu dem für Zwischenprodukt 1, Stufe a) beschriebenen Verfahren. Ausbeute: 5.78 g (91%); Massenspektroskopie [M+H]⁺ = 196.

### b) 3-(4-Fluor-phenyl)-5-methyl-[1,2,4]triazol

Die Darstellung erfolgt in Analogie zur Vorschrift für Zwischenprodukt 1 b) aus 5.77 g (30 mmol) 4-Fluor-benzoesäure-(1-imino-ethyl)-hydrazid. Ausbeute: 4.11 g (78%); Massenspektroskopie [M+H]⁺ = 178.

### c) {3-[3-(4-Fluor-phenyl)-5-methyl-[1,2,4]triazol-1-yl]-1,1-dimethyl-propyl}-carbaminsäure tert-butyl ester

5.88 g (33 mmol) 3-(4-Fluor-phenyl)-5-methyl-[1,2,4]triazol werden in 40 mL DMPU gelöst und in der für Zwischenprodukt 1 c) ausgeführten Weise mit 11.04 g (50 mmol) (3-Chlor-1,1-dimethyl-propyl)-carbaminsäure-tert-butylester, 1.59 g (40 mmol, 60%ig) Natriumhydrid und 2.21 g (6 mmol) Tetrabutylammoniumiodid umgesetzt. Ausbeute: 4.22 g (35%); Massenspektroskopie [M+H]⁺ = 363.

### d) 3-[3-(4-Fluor-phenyl)-5-methyl-[1,2,4]triazol-1-yl]-1,1-dimethyl-propylamin

Erhalten aus der Umsetzung von 4.22 g (11.6 mmol) {3-[3-(4-Fluor-phenyl)-5-methyl-[1,2,4]triazol-1-yl]-1,1-dimethyl-propyl}-carbaminsäure tert-butyl ester in 100 mL Dichlormethan und 15 mL Trifluoressigsäure. Weißer Feststoff. Ausbeute: 4.43 g (Trifluoracetat); Massenspektroskopie [M+H]⁺ = 263.

### Zwischenprodukt 3: 3-[3-(3,5-Difluor-phenyl)-5-methyl-[1,2,4]triazol-1-yl]-1,1-dimethyl-propylamin

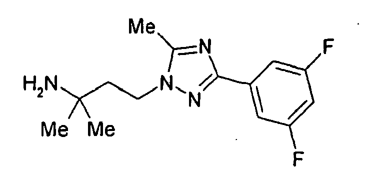

### a) 3,5-Difluor-benzoesäure-(1-imino-ethyl)-hydrazid

Aus 4.91 g (40 mmol) Ethylacetimidat hydrochlorid und 3.80 g (22 mmol) 3,5 Difluorbenzoesäurehydrazid wird die Verbindung in Analogie zur Vorschrift für Zwischenprodukt 1a) erhalten. Ausbeute: 4.49 g (95%); Massenspektroskopie [M+H]⁺ = 214.

### b) 3-(3,5-Difluor-phenyl)-5-methyl-[1,2,4]triazol

Hergestellt aus 4.61 g (22 mmol) 3,5-Difluor-benzoesäure-(1-imino-ethyl)-hydrazid. Ausbeute: 3.81 g (91%); Massenspektroskopie [M+H]⁺ = 196.

### c) {3-[3-(3,5-Difluor-phenyl)-5-methyl-[1,2,4]triazol-1-yl]-1,1-dimethyl-propyl}-carbaminsäure tert-butyl ester

3.74 g (19 mmol) 3-(3,5-Difluor-phenyl)-5-methyl-[1,2,4]triazol in 25 mL DMPU werden mit 0.92 g (23 mmol, 60%ig) Natriumhydrid, 6.37 g (29 mmol) (3-Chlor-1,1-dimethylpropyl)-carbaminsäure-tert-butylester und 1.27 g (3.5 mmol) Tetrabutylammoniumiodid in Analogie zu Beispiel 1c) umgesetzt. Öl. Ausbeute: 2.62 g (36%); Massenspektroskopie [M+H]⁺ = 381.

### d) 3-[3-(3.5-Difluor-phenyl)-5-methyl-[1,2,4]triazol-1-yl]-1,1-dimethyl-propylamin

2.62 g (6.9 mmol) {3-[3-(3,5-Difluor-phenyl)-5-methyl-[1,2,4]triazol-1-yl]-1,1-dimethylpropyl}-carbaminsäure tert-butyl ester in 65 mL Dichlormethan werden mit 9 mL Trifluoressigsäure in der für Zwischenprodukt 1d) beschriebenen Weise umgesetzt. Weißer Feststoff. Ausbeute: 2.11 g (Trifluoracetat); Massenspektroskopie [M+H]⁺ = 281.

### Zwischenprodukt 4: 3-[5-Ethyl-3-(4-methoxy-phenyl)-[1,2,4]triazol-1-yl]-1,1-dimethyl-propylamin

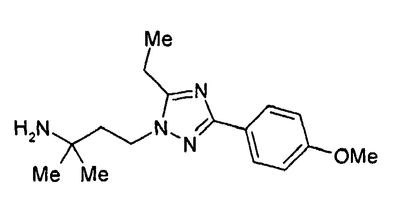

### a) 4-Methoxy-benzoesäure-(1-imino-propyl)-hydrazid

Herstellung aus 4.90 g (45 mmol) Propioamidin Hydrochlorid und 5. 00 g (30 mmol) 4-Methoxy-benzoesäurehydrazid in Analogie zur Vorschrift für Zwischenprodukt 1a). Nach dem Abdestillieren des Ethanols werden 10.0 g Rohprodukt erhalten, die ohne weitere Aufreinigung umgesetzt werden.

### b) 5-Ethyl-3-(4-methoxy-phenyl)-[1,2,4]triazol

9.99 g (60%ig, ca. 28 mmol) 4-Methoxy-benzoesäure-(1-imino-propyl)-hydrazid werden für zwei Stunden auf 150°C erwärmt. Nach Abkühlung wird die Schmelze mittels Chromatographie an einer Kieselgelsäule (Petrolether/Ethylacetat = 3/7) gereinigt. Leicht gelber Feststoff. Ausbeute: 4.56 g (75% über zwei Stufen); Massenspektroskopie [M+H]⁺ = 204.

### c){3-[5-Ethyl-3-(4-methoxy-phenyl)-[1,2,4]triazol-1-yl]-1,1-dimethyl-propylcarbaminsäure tert-butyl ester

4.30 g (21.2 mmol) 5-Ethyl-3-(4-methoxy-phenyl)-[1,2,4]triazol werden in 30 mL DMPU gelöst und auf 0°C gekühlt. Unter Schutzgasatmosphäre werden anschließend portionsweise 1.02 g (24 mmol, 60%ig) Natriumhydrid zugegeben und die Reaktionsmischung wird langsam auf Raumtemperatur erwärmt und dann eine Stunde gerührt. 6.10 g (27.5 mmol) (3-Chlor-1,1-dimethyl-propyl)-carbaminsäure-tert-butylester und 1.41 g (3.8 mmol) Tetrabutylammniumiodid werden zugesetzt. Man läßt über Nacht rühren und beendet dann die Reaktion durch Zugabe von Wasser und Ethylacetat. Die wässrige Phase wird abgetrennt und mit Ethyacetat extrahiert. Die vereinigten organischen Phasen werden mit Natriumchlorid-Lösung gewaschen, mit Natriumsulfat getrocknet und eingeengt. Das zurückbleibende Öl wird mittels Chromatographie an einer Kieselgelsäule (Petrolether/Ethylacetat = 3:7) gereinigt. Ausbeute: 6.82 g (83%); Massenspektroskopie [M+H]⁺ = 389.

### d) 3-[5-Ethyl-3-(4-methoxy-phenyl)-[1,2,4]triazol-1-yl]-1,1-dimethyl-propylamin

Zu einer Lösung von 6.81 g (17.5 mmol) {3-[5-Ethyl-3-(4-methoxy-phenyl)-[1,2,4]triazol-1-yl]-1,1-dimethyl-propyl-carbaminsäure tert-butyl ester in 150 mL Dichlormethan werden insgesamt 20 mL Trifluoressigsäure getropft. Nach drei Stunden Rühren bei Raumtemperatur wird die Lösung eingeengt und das zurückbleibende Öl mit Diethylether versetzt. Der ausfallende weiße Feststoff wird abfiltriert mit Diethylether gewaschen und getrocknet. Ausbeute: 7.86 g (Trifluoracetat); Massenspektroskopie [M+H]⁺ = 289.

### Zwischenprodukt 5: 3-[1-(3-Amino-3-methyl-butyl)-5-methyl-1H-[1,2,4]triazol-3-yl]-benzoesäuremethylester

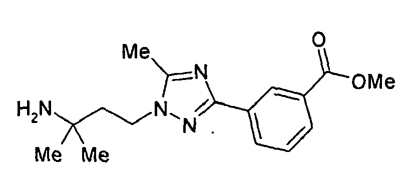

### a) 3-[N'-Benzyloxycarbonyl-hydrazinocarbonyl)-benzoesäuremethylester

Zu einer Lösung von 9.04 g (54.4 mmol) Hydrazincarbonsäurebenzylester in 100 mL Diethylether, 100 mL Dichlormethan und 4.83 mL Pyridin werden unter Kühlung mit einem Eisbad 10.80 g (54.4 mmol) 3-Chlorcarbonyl-benzoesäuremethylester in 100 mL Diethylether getropft. Die Reaktionsmischung wird über Nacht bei Raumtemperatur gerührt und dann mit Wasser versetzt. Der ausfallende Feststoff wird abfiltriert und mit Diethylether gewaschen. Weißer Feststoff. Ausbeute: 14.1 g (79%); Massenspektroskopie [M-H]⁺ = 327.

### b) 3-Hydrazinocarbonyl-benzoesäuremethylester

14.6 g (44.5 mmol) 3-[N'-Benzyloxycarbonyl-hydrazinocarbonyl)-benzoesäuremethylester werden in 75 mL Methanol gelöst und in Gegenwart von Palladium auf Kohle (10%ig) bei Raumtemperatur und 3 bar Wasserstoffdruck hydriert. Der Katalysator wird abfiltriert und das Filtrat vom Lösungsmittel befreit. Weißer Feststoff. Ausbeute: 7.98 g (92%); Massenspektroskopie [M+H]⁺ =195.

### c) 3-[N'-(1-Imino-ethyl)-hydrazinocarbonyl]-benzoesäuremethylester

Hergestellt in Analogie zu dem für Zwischenprodukt 1a) beschriebenen Verfahren aus 3-Hydrazinocarbonyl-benzoesäuremethylester und Ethylacetimidat hydrochlorid. Weißer Feststoff. Ausbeute: 8.60 g (90%); Massenspektroskopie [M+H]⁺ = 236.

### d) 3-(5-Methyl-1H-[1,24]triazol-3-yl)-benzoesäuremethylester

8.10 g (34.4 mmol) 3-[N'-(1-Imino-ethyl)-hydrazinocarbonyl]-benzoesäuremethylester werden für 30 Minuten auf 180°C erwärmt. Zu dem nach Abkühlung vorliegenden Feststoff werden 80 mL Chloroform gegeben. Die Suspension wird filtriert und das Produkt getrocknet. Weißer Feststoff. Ausbeute: 4.03 g (55%); Massenspektroskopie [M+H]⁺ = 218.

### e) 3-[1-(3-tert-Butoxycarbonylamino-3-methyl-butyl)-5-methyl-1H-[1,2,4]triazol-3-yl-benzoesäuremethylester

6.00 g (27.6 mmol) 3-(5-Methyl-1H-[1,24]triazol-3-yl)-benzoesäuremethylester und 9.19 g (41.4 mmol) (3-Chlor-1,1-dimethyl-propyl)-carbaminsäure-tert-butylester werden in der für Zwischenprodukt 1c) beschriebenen Weise umgesetzt und aufgearbeitet. Gelbes Öl. Ausbeute: 5.96 g (54%); Massenspektroskopie [M+H]⁺ = 403.

### f) 3-[1-(-3-Amino-3-methyl-butyl)-5-methyl-1H-[1,2,4]triazol-3-yl]-benzoesäuremethylester

Erhalten aus 3-[1-(3-*tert*-Butoxycarbonylamino-3-methyl-butyl)-5-methyl-1H-[1,2,4]triazol-3-yl-benzoesäuremethylester in Analogie zu dem für Zwischenprodukt 1d) beschriebenen Verfahren. Ausbeute: 5.36 g (68%, Di-trifluoracetat); Massenspektroskopie [M+H]⁺ = 303.

Unter analoger Anwendung vorstehend genannter Syntheseverfahren können ferner die folgenden Zwischenprodukte erhalten werden.

### Zwischenprodukt 6: 3-[5-Methyl-3-(2-methoxy-phenyl)-[1,2,4]triazol-1-yl]-1,1-dimethylpropylamin

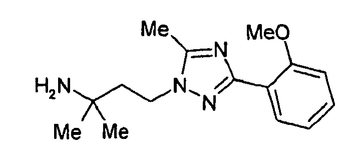

### Zwischenprodukt 7: 3-(5-Methyl-3-(4-methoxy-phenyl)-[1,2,4]triazol-1-yl]-1,1-dimethylpropylamin

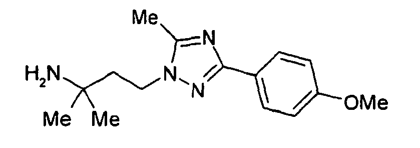

### Zwischenprodukt 8: 3-[5-Methyl-3-(3-benzo[1,3]dioxol-5-yl)-[1,2,4]triazol-1-yl]-1,1-dimethyl-propylamin

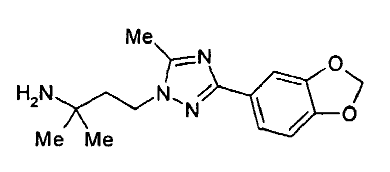

### Zwischenprodukt 9: 2-[3-(4-Methoxy-phenyl)-[1,2,4]triazol-1-yl]-1,1-dimethyl-ethylamin

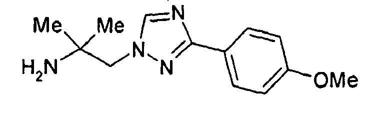

### Zwischenprodukt 10: 1,1,-Dimethyl-3-([1,2,4[triazol-1-yl)-propylamin

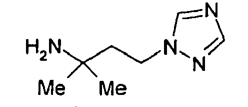

### Allgemeine Arbeitsvorschrift 1 (AAV1):

1 mmol N-[2-Benzyloxy-5-[2-ethoxy-2-hydroxy-acetyl)-phenyl]-methansulfonamid und 1 mmol Amin (bzw. Zwischenprodukt) werden 30 Minuten in 5 mL Tetrahydrofuran bei Raumtemperatur gerührt. Man kühlt auf 0°C ab und tropft unter Argonatmosphäre 1.5 mL einer 2 molaren Lösung von Lithiumborhydrid in Tetrahydrofuran zu. Es wird 15 min bei 0°C gerührt, mit 10 mL Dichlormethan und 3 mL Wasser versetzt, eine weitere Stunde bei Raumtemperatur gerührt und dann über Kieselgur filtriert, wobei man mit Dichlormethan eluiert. Das Eluat wird vom Lösungsmittel befreit und der Rückstand, falls notwendig, chromatographisch gereinigt. Der so erhaltene Benzylether wird in Methanol gelöst und mit Palladium auf Kohle (10%ig) als Katalysator bei 2.5 bar und Raumtemperatur hydriert. Anschließend wird der Katalysator abgetrennt und das Rohprodukt mittels Chromatographie (Reverse Phase, Acetonitril/Wasser-Gradient mit 0.1 % Trifluoressigsäure) gereinigt.

### Beispiel 1: N-[5-(2-{3-[5-Ethyl-3-(4-methoxy-phenyl)-[1,2,4]triazol-1-yl]-1,1-dimethylpropylamino}-1-hydroxy-ethyl)-2-hydroxy-phenyl]-methansulfonamid

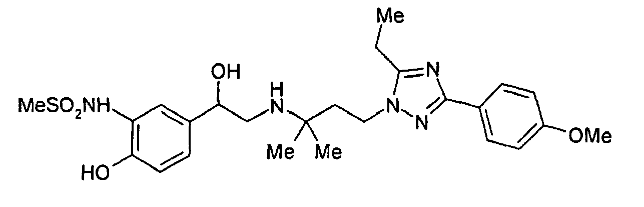

Hergestellt nach der AAV1 aus N-[2-Benzyloxy-5-[2-ethoxy-2-hydroxy-acetyl)-phenyl]-methansulfonamid und 3-[5-Ethyl-3-(4-methoxy-phenyl)-[1,2,4]triazol-1-yl]-1,1-dimethylpropylamin. Ausbeute: 255 mg (40% über 2 Stufen, Trifluoracetat); Massenspektroskopie: [M+H]⁺ = 518.

### Beispiel 2: N-[5-(2-{1,1-Dimethyl-3-[5-methyl-3-(4-trifluoromethyl-phenyl)-[1,2,4]triazol-1-yl]-propylamino}-1-hydroxy-ethyl)-2-hydroxy-phenyl]-methansulfonamid

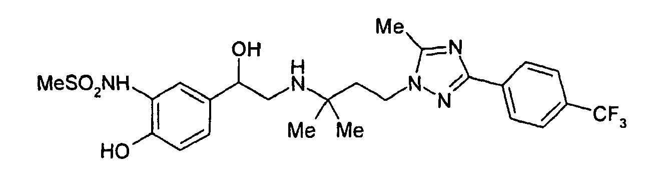

Erhalten nach der AAV1 aus der Umsetzung von N-[2-Benzyloxy-5-[2-ethoxy-2-hydroxyacetyl)-phenyl]-methansulfonamid und 1,1-Dimethyl-3-[5-methyl-3-(4-trifluormethylphenyl)-[1,2,4]triazol-1-yl]-propylamin. Weißer Feststoff. Ausbeute: 78 mg (12% über 2 Stufen, Trifluoracetat); Massenspektroskopie: [M+H]⁺ = 541.

### Beispiel 3: N-(5-{2-1,1-Dimethyl-3-(5-methyl-3-p-tolyl-[1,2,4]triazol-1-yl)-propylamino]-1-hydroxy-ethyl}-2-hydroxy-phenyl)-methansulfonamid

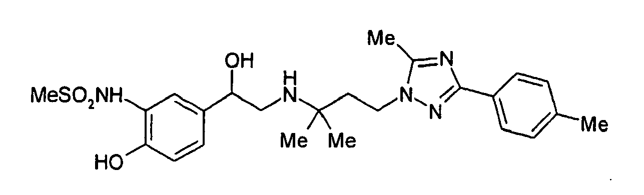

Erhalten nach der AAV1 aus N-[2-Benzyloxy-5-[2-ethoxy-2-hydroxy-acetyl)-phenyl]-methansulfonamid und 1,1-Dimethyl-3-(5-methyl-3-p-tolyl-[1,2,4]triazol-1-yl)-propylamin. Weißer Feststoff. Ausbeute: 7 mg (1% über 2 Stufen, Trifluoracetat); Massenspektroskopie: [M+H]⁺ = 488.

### Beispiel 4: N-[5-(2-{3-[3-(4-Fluoro-phenyl)-5-methyl-[1,2,4]triazol-1-yl)-1,1-dimethylpropylamino)-1-hydroxy-ethyl)-2-hydroxy-phenyl]-methansulfonamid

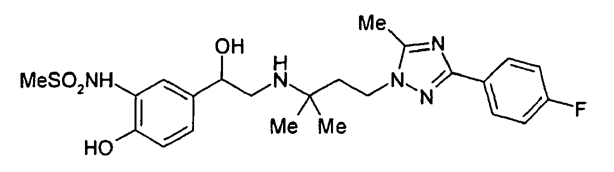

Herstellung aus N-[2-Benzyloxy-5-[2-ethoxy-2-hydroxy-acetyl)-phenyl]-methansulfonamid und 3-[3-(4-Fluor-phenyl)-5-methyl-[1,2,4]triazol-1-yl]-1,1-dimethylpropylamin nach der AAV1. Weißer Feststoff. Ausbeute: 155 mg (26% über 2 Stufen, Trifluoracetat); Massenspektroskopie: [M+H]⁺ = 492.

### Beispiel 5: 3-(1-{3-[2-Hydroxy-2-(4-hydroxy-3-methansulfonylamino-phenyl)-ethylamino]-3-methyl-bulyl}-5-methyl-1H-[1,2,4]triazol-3-yl)-benzoesäuremethylester

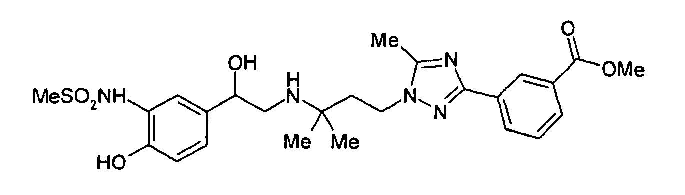

Herstellung nach der AAV1aus N-[2-Benzyloxy-5-[2-ethoxy-2-hydroxy-acetyl)-phenyl]-methansulfonamid und 3-[1-(3-Amino-3-methyl-butyl)-5-methyl-1H-[1,2,4]triazol-3-yl]-benzoesäuremethylester. Weißer Feststoff. Ausbeute: 36 mg (7% über 2 Stufen, Trifluoracetat); Massenspektroskopie: [M+H]⁺ = 532.

### Beispiel 6: N-[5-(2-{3-[3-(3,5-Difluor-phenyl)-5-methyl-[1,2,4]triazol-1-yl]-1,1-dimethylpropylamino}-1-hydroxy-ethyl)-2-hydroxy-phenyl]-methansulfonamid

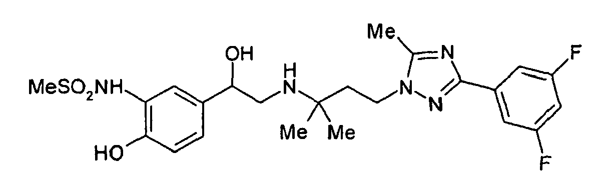

Herstellung nach der AAV1 aus N-[2-Benzyloxy-5-[2-ethoxy-2-hydroxy-acetyl)-phenyl]-methansulfonamid und 3-[3-(3,5-Difluor-phenyl)-5-methyl-[1,2,4]triazol-1-yl]-1,1-dimethyl-propylamin. Weißer Feststoff. Ausbeute: 20 mg (3% über 2 Stufen, Trifluoracetat); Massenspektroskopie: [M+H]⁺ = 510.

### Beispiel 7: N-(2-Hydroxy-5-(1-hydroxy-2-{3-[3-(2-methoxy-phenyl)-5-methyl-[1,2,4]triazol-1-yl]-1,1-dimethyl-propylamino}-ethyl)-phenyl]-methansulfonamid

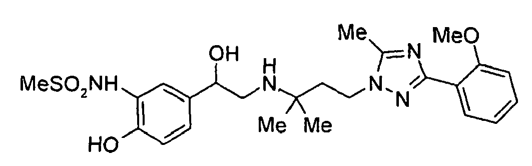

347 mg (1 mmol) 3-[3-(2-Methoxy-phenyl)-5-methyl-[1,2,4]triazol-1-yl]-1,1-dimethylpropylamin Hydrochlorid werden mit Natriumhydroxid-Lösung versetzt und 2 Stunden bei Raumtemperatur gerührt. Die Lösung wird auf Kieselgur gegeben und mit Dichlormethan eluiert. Das Eluat wird eingeengt und der Rückstand in 5 mL THF aufgenommen. Es werden 379 mg (1 mmol) *N*-[2-Benzyloxy-5-[2-ethoxy-2-hydroxy-acetyl)-phenyl]-methansulfonamid zugegeben und man läßt 30 min bei Raumtemperatur rühren. Nach Abkühlung auf 0°C werden 1.5 mL einer 2 molaren Lösung von Lithiumborhydrid in THF zugetropft und es wird 30 Minuten bei Raumtemperatur gerührt. Die Reaktionsmischung wird mit 10 mL Dichlormethan und 3 mL Wasser versetzt, eine Stunde gerührt und dann mit Dichlormethan als Eluens über Kieselgur filtriert. Das Lösungsmittel wird abdestilliert und der Rückstand in 5 mL Methanol aufgenommen. Anschließend wird mit 100 mg Palladium auf Kohle bei 2.5 bar hydriert. Der Katalysator wird abgetrennt und das Filtrat eingeengt. Zur weiteren Reinigung wird der Rückstand chromatographiert (RP, Acetonitril:Wasser-Gradient mit 0.1 % Trifluoracetat). Ausbeute: 323 mg (52%, Trifluoracetat); Massenspektrometrie: [M+H]⁺ = 504.

### Beispiel 8: N-(2-Hydroxy-5-(1-hydroxy-2-{3-[3-(4-methoxy-phenyl)-5-methyl-[1,2,4]triazol-1-yl]-1,1-dimethyl-propylamino}-ethyl)-phenyl]-methansulfonamid

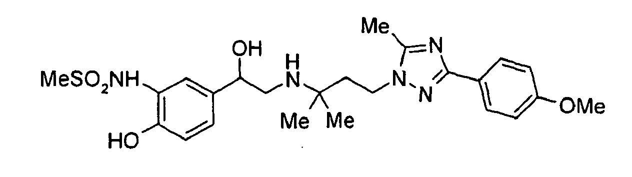

379 mg (1 mmol) *N*-[2-Benzyloxy-5-[2-ethoxy-2-hydroxy-acetyl)-phenyl]-methansulfonamid und 274 mg (1 mmol) 3-[3-(4-Methoxy-phenyl)-5-methyl-[1,2,4]triazol-1-yl]-1,1-dimethyl-propylamin werden in 5 mL Ethanol suspendiert und auf 70°C erwärmt. Die entstandene Lösung wird eine Stunde bei 70°C gerührt und dann auf Raumtemperatur abgekühlt. Nach Zugabe von 113 mg (3 mmol) Natriumborhydrid wird 3 Stunden bei Raumtemperatur gerührt, mit 0.7 mL gesättigter Kaliumcarbonatlösung versetzt und weitere 30 Minuten gerührt. Es wird über Aluminiumoxid (basisch) filtriert, wiederholt mit Methylenchlorid/Methanol 15:1 nachgewaschen, eingeengt und chromatographiert (Kieselgel; Dichlormethan mit 0-10% Methanol:Ammoniak = 9:1). Die so erhaltene Benzylverbindung wird in 10 mL Methanol gelöst und mit Palladium auf Kohle bei 2.5 bar Wasserstoffdruck hydriert. Anschließend wird filtriert und das Filtrat eingeengt. Ausbeute: 339 mg (67%); Massenspektrometrie: [M+H]⁺ = 504.

### Beispiel 9: N-(5-{2-[3-(3-Benzo[1,3]dioxol-5-yl-5-methyl-[1,2,4]triazol-1-yl)-1,1-dimethyl-propylaminol-1-hydroxy-ethyl}-2-hydroxyphenyl)-methansulfonamid

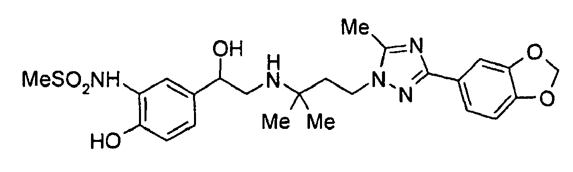

In Analogie zur Vorschrift für Beispiel 7 werden 379 mg (1 mmol) *N*-[2-Benzyloxy-5-[2-ethoxy-2-hydroxy-acetyl)-phenyl]-methansulfonamid und 288 mg (1 mmol) 3-(3-Benzo[1,3]dioxol-5-yl-5-methyl-[1,2,4]triazol-1-yl)-1,1-dimethyl-propylamin miteinander umgesetzt. Die anschließende Debenzylierung liefert die Zielverbindung. Ausbeute: 371 mg (72%); Massenspektrometrie: [M+H]⁺ = 518.

### Beispiel 10: N-[2-Hydroxy-5-(1-hydroxy-2-{3-[3-(4-methoxy-phenyl)-[1,2,4]triazo1-1-yl]-1,1-dimethyl-propylamino}-ethyl)-phenyl]-methansulfonamid

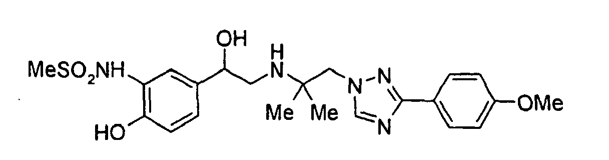

Aus der Umsetzung von 379 mg (1 mmol) *N*-[2-Benzyloxy-5-[2-ethoxy-2-hydroxyacetyl)-phenyl]-methansulfonamid und 246 mg (1 mmol) 2-[3-(4-Methoxy-phenyl)-[1,2,4]triazol-1-yl]-1,1-dimethyl-ethylamin in der für Beispiel 7 beschriebenen Weise und nachfolgender Debenzylierung wird die Zielverbindung erhalten. Ausbeute: 305 mg (64%); Massenspektrometrie: [M+H]⁺ = 476.

### Beispiel 11: N-{5-[2-[1,1-Dimethyl-3-[1,2,4]triazol-1-yl-propylamino)-1-hydroxy-ethyl]-2-hydroxy-phenyl}-methanesulfonamid

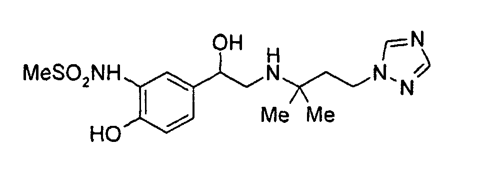

Die Zielverbindung wird in Analogie zu den Vorschriften für Beispiel 7 aus 379 mg (1 mmol) *N*-[2-Benzyloxy-5-[2-ethoxy-2-hydroxy-acetyl)-phenyl]-methanesulfonamid und 154 mg (1 mmol) 1,1-Dimethyl-3-[1,2,4]triazol-1-yl-propylamin hergestellt. Farbloser Feststoff. Ausbeute: 225 mg (59%); Massenspektrometrie: [M+H]⁺ = 384.

Wie gefunden wurde, zeichnen sich die Verbindungen der Formel **1** durch vielfältige Anwendungsmöglichkeiten auf therapeutischem Gebiet aus. Hervorzuheben sind solche Anwendungsmöglichkeiten, für welche die erfindungsgemäßen Verbindungen der Formel **1** aufgrund ihrer pharmazeutischen Wirksamkeit als Betamimetikum bevorzugt zur Anwendung gelangen können.

Dies sind beispielsweise die Behandlung von entzündlichen und obstruktiven Atemwegserkrankungen, die ausgewählt sind aus der Gruppe bestehend aus Obstruktive Lungenerkrankungen unterschiedlicher Genese, Lungenemphyseme unterschiedlicher Genese, Restriktive Lungenerkrankungen, Interstitielle Lungenerkrankungen, Zystische Fibrose, Bronchitiden unterschiedlicher Genese, Bronchiektasen, ARDS (adult respiratory distress syndrom) und alle Formen des Lungenödems.

Bevorzugt werden die Verbindungen der Formel **1** zur Herstellung eines Arzneimittels zur Behandlung von obstruktiven Lungenerkrankungen verwendet, die ausgewählt sind aus der Gruppe bestehend aus Asthma Bronchiale, pädiatrisches Asthma, schweres Asthma, akuter Asthma-Anfall, chronische Bronchitis und COPD (chronisch obstruktive pulmonale Erkrankung), wobei die Verwendung zur Herstellung eines Arzneimittels zur Behandlung von Asthma Bronchiale und COPD erfindungsgemäß besonders bevorzugt ist.

Bevorzugt werden die Verbindungen der Formel **1** ferner zur Herstellung eines Arzneimittels zur Behandlung von Lungenemphysemen verwendet, die ihren Ursprung haben in COPD oder α1-Proteinase-Inhibitor-Mangel.

Bevorzugt werden die Verbindungen der Formel **1** ferner zur Herstellung eines Arzneimittels zur Behandlung von Restriktiven Lungenerkrankungen verwendet, die ausgewählt sind aus der Gruppe bestehend aus Allergische Alveolitis, durch berufliche Noxen ausgelöste restriktive Lungenerkrankungen wie Asbestose oder Silikose und Restriktion aufgrund von Lungentumoren, wie beispielsweise Lymphangiosis carcinomatosa, bronchoalveoläres Karzinom und Lymphome.

Bevorzugt werden die Verbindungen der Formel **1** ferner zur Herstellung eines Arzneimittels zur Behandlung von Interstitiellen Lungenerkrankungen verwendet, die ausgewählt sind aus der Gruppe bestehend aus infektiös bedingte Pneumonien, wie beispielsweise aufgrund einer Infektion mit Viren, Bakterien, Pilzen, Protozoen, Helminthen oder anderen Erregern, Pneumonitis aufgrund unterschiedlicher Genese, wie beispielsweise Aspiration und Linksherzinsuffizienz, Strahlen-induzierte Pneumonitis oder Fibrose, Kollagenosen, wie beispielsweise Lupus erythematodes, sytemische Sklerodermie oder Sarkoidose, Granulomatosen, wie beispielsweise Morbus Boeck, idiopathische interstitielle Pneumonie oder idiopathische pulmonäre Fibrose (IPF).

Bevorzugt werden die Verbindungen der Formel **1** ferner verwendet zur Herstellung eines Arzneimittels zur Behandlung von Zystischer Fibrose bzw. Mukoviszidose.

Bevorzugt ist ferner Verwendung der Verbindungen der Formel **1** zur Herstellung eines Arzneimittels zur Behandlung von Bronchitiden, wie beispielsweise Bronchitis aufgrund bakterieller oder viraler Infektion, Allergische Bronchitis und Toxische Bronchitis.

Bevorzugt ist ferner Verwendung der Verbindungen der Formel **1** zur Herstellung eines Arzneimittels zur Behandlung von Bronchiektasen.

Bevorzugt ist ferner Verwendung der Verbindungen der Formel 1 zur Herstellung eines Arzneimittels zur Behandlung von ARDS (adult respiratory distress syndrom).

Bevorzugt ist ferner Verwendung der Verbindungen der Formel **1** zur Herstellung eines Arzneimittels zur Behandlung von Lungenödemen, beispielsweise toxischer Lungenödeme nach Aspiration oder Inhalation von toxischen Substanzen und Fremdstoffen.

Besonders bevorzugt ist ferner Verwendung der Verbindungen der Formel **1** zur Herstellung eines Arzneimittels zur Behandlung von Asthma oder COPD. Von besonderer Bedeutung ist ferner die vorstehend genannte Verwendung zur Herstellung eines Arzneimittels zur einmal täglichen Behandlung von entzündlichen und obstruktiven Atemwegserkrankungen, besonders bevorzugt zur einmal täglichen Behandlung von Asthma oder COPD.

Geeignete Anwendungsformen zur Applikation der Verbindungen der Formel **1** sind beispielsweise Tabletten, Kapseln, Zäpfchen, Lösungen, Pulver etc. Der Anteil der pharmazeutisch wirksamen Verbindung(en) sollte jeweils im Bereich von 0,05 bis 90 Gew.-%, bevorzugt 0,1 bis 50 Gew.-% der Gesamtzusammensetzung liegen. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Lösungen werden in üblicher Weise, z.B. unter Zusatz von Isotonantien, Konservierungsmitteln, wie p-Hydroxybenzoaten, oder Stabilisatoren, wie Alkalisalzen der Ethylendiamintetraessigsäure, gegebenenfalls unter Verwendung von Emulgiermitteln und /oder Dispergiermitteln, wobei beispielsweise bei der Verwendung von Wasser als Verdünnungsmittel gegebenenfalls organische Lösungsmittel als Lösevermittler bzw. Hilfslösungsmittel eingesetzt werden können, hergestellt und in Injektionsflaschen oder Ampullen oder Infusionsflaschen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägem, wie Milchzucker oder Sorbit, mischt und in Gelatinekapseln einkapselt. Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Als Hilfsstoffe seien beispielsweise Wasser, pharmazeutisch unbedenkliche organische Lösungsmittel, wie Paraffine (z.B. Erdölfraktionen), Öle pflanzlichen Ursprungs (z.B. Erdnuss- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker), Emulgiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mit verwendet werden. Im Falle wässriger Suspensionen können die Wirkstoffe außer den oben genannten Hilfsstoffen mit verschiedenen Geschmacksaufbesserern oder Farbstoffen versetzt werden.

Bei der erfindungsgemäß bevorzugten Applikation der Verbindungen der Formel **1** zur Therapie von Asthma oder COPD werden besonders bevorzugt inhalativ applizierbare Darreichungsformen bzw. pharmazeutische Formulierungen eingesetzt. Als inhalierbare Darreichungsformen kommen Inhalationspulver, treibgashaltige Dosieraerosole oder treibgasfreie Inhalationslösungen in Betracht. Im Rahmen der vorliegenden Erfindung sind von dem Begriff treibgasfreie Inhalationslösungen auch Konzentrate oder sterile, gebrauchsfertige Inhalationslösungen umfasst. Die im Rahmen der vorliegenden Erfindung einsetzbaren Darreichungsformen werden im nachfolgenden Teil der Beschreibung detailliert beschrieben.

Erfindungsgemäß einsetzbare Inhalationspulver können **1** entweder allein oder im Gemisch mit geeigneten physiologisch unbedenkliche Hilfsstoffen enthalten.

Sind die Wirkstoffe **1** im Gemisch mit physiologisch unbedenklichen Hilfsstoffen enthalten, können zur Darstellung dieser erfindungsgemäßen Inhalationspulver die folgenden physiologisch unbedenklichen Hilfsstoffe zur Anwendung gelangen: Monosaccharide (z.B. Glucose oder Arabinose), Disaccharide (z.B. Lactose, Saccharose, Maltose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt Lactose, höchst bevorzugt Lactosemonohydrat als Hilfsstoff zur Anwendung.

Die Hilfsstoffe weisen im Rahmen der erfindungsgemäßen Inhalationspulver eine maximale mittlere Teilchengröße von bis zu 250µm, bevorzugt zwischen 10 und 150µm, besonders bevorzugt zwischen 15 und 80µm auf. Gegebenenfalls kann es sinnvoll erscheinen, den vorstehend genannten Hilfsstoffen feinere Hilfsstofffraktionen mit einer mittleren Teilchengröße von 1 bis 9µm beizumischen. Letztgenannte feinere Hilfsstoffe sind ebenfalls ausgewählt aus der vorstehend genannten Gruppe an einsetzbaren Hilfsstoffen. Schließlich wird zur Herstellung der erfindungsgemäßen Inhalationspulver mikronisierter Wirkstoff **1**, vorzugsweise mit einer mittleren Teilchengröße von 0,5 bis 10µm, besonders bevorzugt von 1 bis 5µm, der Hilfsstoffmischung beigemischt. Verfahren zur Herstellung der erfindungsgemäßen Inhalationspulver durch Mahlen und Mikronisieren sowie durch abschließendes Mischen der Bestandteile sind aus dem Stand der Technik bekannt. Die erfindungsgemäßen Inhalationspulver können mittels aus dem Stand der Technik bekannten Inhalatoren appliziert werden.

Erfindungsgemäße treibgashaltige Inhalationsaerosole können **1** im Treibgas gelöst oder in dispergierter Form enthalten. Hierbei können **1** in getrennten Darreichungsformen oder in einer gemeinsamen Darreichungsform enthalten sein, wobei **1** entweder beide gelöst, beide dispergiert oder jeweils nur eine Komponente gelöst und die andere dispergiert enthalten sein können.

Die zur Herstellung der Inhalationsaerosole einsetzbaren Treibgase sind aus dem Stand der Technik bekannt. Geeignete Treibgase sind ausgewählt aus der Gruppe bestehend aus Kohlenwasserstoffen wie *n*-Propan, *n*-Butan oder Isobutan und Halogenkohlenwasserstoffen wie fluorierten Derivaten des Methans, Ethans, Propans, Butans, Cyclopropans oder Cyclobutans. Die vorstehend genannten Treibgase können dabei allein oder in Mischungen derselben zur Verwendung kommen. Besonders bevorzugte Treibgase sind halogenierte Alkanderivate ausgewählt aus TG134a und TG227 und Mischungen derselben.

Die treibgashaltigen Inhalationsaerosole können ferner weitere Bestandteile wie Kosolventien, Stabilisatoren, oberflächenaktive Mittel (surfactants), Antioxidantien, Schmiermittel sowie Mittel zur Einstellung des pH-Werts enthalten. All diese Bestandteile sind im Stand der Technik bekannt.

Die vorstehend genannten treibgashaltigen Inhalationaerosole können mittels im Stand der Technik bekannten Inhalatoren (MDIs = metered dose inhalers) appliziert werden.

Ferner kann die Applikation der erfindungsgemäßen Wirkstoffe **1** in Form von treibgasfreien Inhalationslösungen und Inhalationssuspensionen erfolgen. Als Lösungsmittel kommen hierzu wässrige oder alkoholische, bevorzugt ethanolische Lösungen in Betracht. Das Lösungsmittel kann ausschließlich Wasser sein oder es ist ein Gemisch aus Wasser und Ethanol. Der relative Anteil an Ethanol gegenüber Wasser ist nicht begrenzt, bevorzugt liegt die maximale Grenze jedoch bei bis 70 Volumenprozent, insbesondere bei bis zu 60 Volumenprozent und besonders bevorzugt bei bis zu 30 Volumenprozent. Die restlichen Volumenprozente werden von Wasser aufgefüllt. Die **1** enthaltenden Lösungen oder Suspensionen werden mit geeigneten Säuren auf einen pH-Wert von 2 bis 7, bevorzugt von 2 bis 5 eingestellt. Zur Einstellung dieses pH-Werts können Säuren ausgewählt aus anorganischen oder organischen Säuren Verwendung finden. Beispiele für besonders geeignete anorganische Säuren sind Salzsäure, Bromwasserstoffsäure, Salpetersäure, Schwefelsäure und/oder Phosphorsäure. Beispiele für besonders geeignete organische Säuren sind: Ascorbinsäure, Zitronensäure, Äpfelsäure, Weinsäure, Maleinsäure, Bernsteinsäure, Fumarsäure, Essigsäure, Ameisensäure und/oder Propionsäure und andere. Bevorzugte anorganische Säuren sind Salzsäure und Schwefelsäure. Es können auch die Säuren verwendet werden, die bereits mit einem der Wirkstoffe ein Säureadditionssalz bilden. Unter den organischen Säuren sind Ascorbinsäure, Fumarsäure und Zitronensäure bevorzugt. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe, Antioxidantien oder Komplexbildner besitzen, wie beispielsweise Zitronensäure oder Ascorbinsäure. Erfindungsgemäß besonders bevorzugt wird Salzsäure zur Einstellung des pH-Werts verwendet.

In diesen Formulierungen kann gegebenenfalls auf den Zusatz von Editinsäure (EDTA) oder einem der bekannten Salze davon, Natriumedetat, als Stabilisator oder Komplexbildner verzichtet werden. Andere Ausführungsformen beinhalten diese Verbindung(en). In einer solchen bevorzugten Ausführungsform liegt der Gehalt bezogen auf Natriumedetat unter 100 mg / 100 ml, bevorzugt unter 50 mg/ 100ml, besonders bevorzugt unter 20 mg/ 100ml. Generell sind solche Inhalationslösungen bevorzugt, in denen der Gehalt an Natriumedetat bei 0 bis 10mg/100ml liegt.

Den treibgasfreien Inhalationslösungen können Co-Solventien und/oder weitere Hilfsstoffe zugesetzt werden. Bevorzugte Co-Solventien sind solche, die Hydroxylgruppen oder andere polare Gruppen enthalten, beispielsweise Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester. Unter Hilfs- und Zusatzstoffen wird in diesem Zusammenhang jeder pharmakologisch verträgliche Stoff verstanden, der kein Wirkstoff ist, aber zusammen mit dem (den) Wirkstoff(en) in dem pharmakologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. oberflächenaktive Stoffe, wie z.B. Sojalecithin, Ölsäure, Sorbitanester, wie Polysorbate, Polyvinylpyrrolidon sonstige Stabilisatoren, Komplexbildner, Antioxidantien und/oder Konservierungsstoffe, die die Verwendungsdauer der fertigen Arzneimittelformulierung gewährleisten oder verlängern, Geschmackstoffe, Vitamine und/oder sonstige dem Stand der Technik bekannte Zusatzstoffe. Zu den Zusatzstoffen zählen auch pharmakologisch unbedenkliche Salze wie beispielsweise Natriumchlorid als Isotonantien.

Zu den bevorzugten Hilfsstoffen zählen Antioxidantien, wie beispielsweise Ascorbinsäure, sofern nicht bereits für die Einstellung des pH-Werts verwendet, Vitamin A, Vitamin E, Tocopherole und ähnliche im menschlichen Organismus vorkommende Vitamine oder Provitamine.

Konservierungsstoffe können eingesetzt werden, um die Formulierung vor Kontamination mit Keimen zu schützen. Als Konservierungsstoffe eignen sich die dem Stand der Technik bekannten, insbesondere Cetylpyridiniumchlorid, Benzalkoniumchlorid oder Benzoesäure bzw. Benzoate wie Natriumbenzoat in der aus dem Stand der Technik bekannten Konzentration. Die vorstehend genannten Konservierungsstoffe sind vorzugsweise in Konzentrationen von bis zu 50mg/100ml, besonders bevorzugt zwischen 5 und 20 mg/100ml enthalten.

Bevorzugte Formulierungen enthalten außer dem Lösungsmittel Wasser und dem Wirkstoff **1** nur noch Benzalkoniumchlorid und Natriumedetat.

In einer anderen bevorzugten Ausführungsform wird auf Natriumedetat verzichtet.

Die Dosierung der erfindungsgemäßen Verbindungen ist naturgemäß stark von der Applikationsart und der zu therapierenden Erkrankung abhängig. Bei inhalativer Applikation zeichnen sich die Verbindungen der Formel **1** bereits bei Dosen im µg-Bereich durch eine hohe Wirksamkeit aus. Auch oberhalb des µg-Bereichs, lassen sich die Verbindungen der Formel **1** sinnvoll einsetzen. Die Dosierung kann dann beispielsweise auch im Milligrammbereich liegen.

Ein weiterer Aspekt der vorliegenden Erfindung betrifft die vorstehend genannten pharmazeutischen Formulierungen als solche, die durch einen Gehalt an einer Verbindung der Formel **1** gekennzeichnet sind, besonders bevorzugt die vorstehend genannten inhalativ applizierbaren pharmazeutischen Formulierungen.

Die nachfolgenden Formulierungsbeipiele illustrieren die vorliegende Erfindung ohne sie jedoch in ihrem Umfang zu beschränken:

| A) | Tabletten | pro Tablette |
|---|---|---|
| | Wirkstoff der Formel **1** | 100 mg |
| | Milchzucker | 140 mg |
| | Maisstärke | 240 mg |
| | Polyvinylpyrrolidon | 15 mg |
| | Magnesiumstearat | 5 mg |
| | | |
| | | 500 mg |

Der feingemahlene Wirkstoff, Milchzucker und ein Teil der Maisstärke werden miteinander vermischt. Die Mischung wird gesiebt, worauf man sie mit einer Lösung von Polyvinylpyrrolidon in Wasser befeuchtet, knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Größe verpresst.

| B) | Tabletten | pro Tablette |
|---|---|---|
| | Wirkstoff der Formel **1** | 80 mg |
| | Milchzucker | 55 mg |
| | Maisstärke | 190 mg |
| | Mikrokristalline Cellulose | 35 mg |
| | Polyvinylpyrrolidon | 15 mg |
| | Natrium-carboxymethylstärke | 23 mg |
| | Magnesiumstearat | 2 mg |
| | | |
| | | 400 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natriumcarboxymethylstärke und das Magnesiumstearat, vermischt und verpresst das Gemisch zu Tabletten geeigneter Größe.

| C) | Ampullenlösung | |
|---|---|---|
| | Wirkstoff der Formel **1** | 50 mg |
| | Natriumchlorid | 50 mg |
| | Aqua pro inj. | 5 ml |

Der Wirkstoff wird bei Eigen-pH oder gegebenenfalls bei pH 5,5 bis 6,5 in Wasser gelöst und mit Natriumchlorid als Isotonans versetzt. Die erhaltene Lösung wird pyrogenfrei filtriert und das Filtrat unter aseptischen Bedingungen in Ampullen abgefühlt, die anschließend sterilisiert und zugeschmolzen werden. Die Ampullen enthalten 5 mg, 25 mg und 50 mg Wirkstoff.

| D) | Dosieraerosol | | |
|---|---|---|---|
| | Wirkstoff der Formel **1** | | 0,005 |
| | Sorbitantrioleat | | 0,1 |
| | Monofluortrichlormethan und | | |
| | TG134a : TG227 2:1 | ad | 100 |

Die Suspension wird in einen üblichen Aerosolbehälter mit Dosierventil gefüllt. Pro Betätigung werden vorzugsweise 50 µl Suspension abgegeben. Der Wirkstoff kann gewünschtenfalls auch höher dosiert werden (z.B. 0.02 Gew.-%).

| | | | |
|---|---|---|---|
| F) | Inhalationspulver | | |
| | Wirkstoff der Formel **1** | | 12 µg |
| | Lactose Monohydrat | ad | 10 mg |

Die Herstellung des Inhalationspulvers erfolgt in üblicher Art und Weise durch Mischen der einzelnen Bestandteile.

## Patentansprüche

1. Verwendung von Verbindungen der Formel **1** 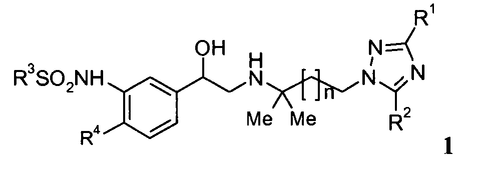 worin
R¹ Wasserstoff, -C₁₋₆-alkyl, -C₁₋₆-haloalkyl, -OH, -O-C₁₋₆-alkyl, Halogen oder ein Rest ausgewählt aus der Gruppe Aryl oder Heterocyclus, wobei Aryl oder Heterocyclus, wenn möglich jeweils optional substituiert mit 1, 2, 3, 4 oder 5 gleichen oder unterschiedlichen Resten R⁵ sind;
R² Methyl;
R³ -C₁₋₆-alkyl;
R⁴ -OH;
R⁵ Halogen, -CN, -NO₂, -C₁₋₆-alkyl, -C₃₋₆-cycloalkyl, -C₁₋₆-haloalkyl, -COR⁶, -COOR⁶, -CONR⁶R⁷, -OR⁶, -NR⁶R⁷, -NR⁶COR⁷, -NR⁶SO₂R⁷, -SR⁶, -SOR⁶, -SO₂R⁶ oder -SO₂NR⁶R⁷, oder zwei R⁵ miteinander verbunden eine Gruppe ausgewählt aus -C₂₋₆-alkylen, -C₂₋₆-alkenylen und -O-C₁₋₆-alkylen-O-;
R⁶ und R⁷ Wasserstoff, -C₁₋₆-alkyl, -C₃₋₆-cycloalkyl;
n 0, 1, 2 oder 3;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren zur Herstellung eines Arzneimittels zur Behandlung von entzündlichen und obstruktiven Atemwegserkrankungen

2. Verwendung von Verbindungen der Formel **1** nach Anspruch 1, worin R², R³, R⁴ und n die oben genannte Bedeutung haben und
R¹ Wasserstoff, -C₁₋₆-alkyl, -C₁₋₆-haloalkyl, -OH, -O-C₁₋₆-alkyl, Halogen oder Aryl, wenn möglich optional substituiert mit 1, 2, 3, 4 oder 5 gleichen oder unterschiedlichen Resten R⁵;
R⁵ Halogen, -CN, -NO₂, -C₁₋₆-alkyl, -C₃₋₆-cycloalkyl, -C₁₋₆-haloalkyl, -COR⁶, -COOR⁶, -CONR⁶R⁷ -OR⁶, -NR⁶R⁷, -NR⁶COR⁷, -NR⁶SO₂R⁷, -SR⁶, -SOR⁶, -SO₂R⁶ oder -SO₂NR⁶R⁷, oder zwei R⁵ miteinander verbunden eine Gruppe ausgewählt aus -C₂₋₆-alkylen, -C₂₋₆-alkenylen und -O-C₁₋₆-alkylen-O-;
R⁶ und R⁷ Wasserstoff, -C₁₋₆-alkyl, -C₃₋₆-cycloalkyl;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

3. Verwendung von Verbindungen der Formel **1** nach einem der Ansprüche 1 oder 2,
worin
R¹ Wasserstoff, -C₁₋₆-alkyl, -O-C₁₋₆-alkyl, Aryl, wenn möglich optional substituiert mit 1, 2, 3, 4 oder 5 gleichen oder unterschiedlichen Resten R⁵;
R² Methyl;
R³ Methyl;
R⁴ -OH;
R⁵ Halogen, -CN, -NO₂, -C₁₋₆-alkyl, -C₃₋₆-cycloalkyl, -C₁₋₆-haloalkyl, -COR⁶, -CO0R⁶, -CONR⁶R⁷ -OR⁶, -NR⁶R⁷, -NR⁶COR⁷, -NR⁶SO₂R⁷, -SR⁶, -SOR⁶, -SO₂R⁶ oder -SO₂NR⁶R⁷, oder zwei R⁵ miteinander verbunden bedeuten eine Gruppe ausgewählt aus -C₂₋₆-alkylen, -C₂₋₆-alkenylen und -O-C₁₋₆-alkylen-O-;
R⁶ und R⁷ Wasserstoff, -C₁₋₆-alkyl, -C₃₋₆-cycloalkyl;
n 0, 1, 2 oder 3;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

4. Verwendung von Verbindungen der Formel **1** nach einem der Ansprüche 1-3, worin
R¹ Wasserstoff, Aryl, wenn möglich optional substituiert mit 1, 2, 3, 4 oder 5 gleichen oder unterschiedlichen Resten R⁵;
R² Methyl;
R³ Methyl;
R⁴ -OH;
R⁵ Halogen, -CN, -NO₂, -C₁₋₆-alkyl, -C₃₋₆-cycloalkyl, -C₁₋₆-haloalkyl, -COR⁶, -COOR⁶, -CONR⁶R⁷, -OR⁶, -NR⁶R⁷, -NR⁶COR⁷, -NR⁶SO₂R⁷, -SR⁶, -SOR⁶, -SO₂R⁶ oder -SO₂NR⁶R⁷, oder zwei R⁵ miteinander verbunden bedeuten eine Gruppe ausgewählt aus -C₂₋₆-alkylen, -C₂₋₆-alkenylen und -O-C₁₋₆-alkylen-O-;
R⁶ und R⁷ Wasserstoff, -C₁₋₆-alkyl, -C₃₋₆-cyctoatkyl;
n 0, 1, 2 oder 3;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

5. Verwendung nach einem der Ansprüche 1-4, **dadurch gekennzeichnet, dass** die Verbindungen der Formel **1** in Form ihrer R-Enantiomere zur Anwendung gelangen.

6. Verbindungen der Formel **1** 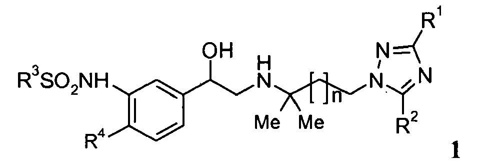 worin R¹ und n die in den Ansprüchen 1-4 genannten Bedeutungen haben können und worin
R² Methyl oder Ethyl;
R³ Methyl;
R⁴ OH,
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

7. Verbindungen der Formel **1** nach Anspruch 6, worin n = 1 bedeutet, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

8. Verbindungen der Formel **1** nach einem der Ansprüche 6 oder 7, worin
R¹ Wasserstoff, Phenyl, optional substituiert mit 1, 2 oder 3 gleichen oder unterschiedlichen Resten R⁵;
R² Methyl oder Ethyl;
R³ Methyl;
R⁴ OH;
R⁵ Halogen, -C₁₋₆-alkyl, -C₃₋₆-cycloalkyl, -C₁₋₆-haloalkyl, -COOR⁶, -CONR⁶R⁷, -OR⁶, -NR⁶R⁷, -NR⁶COR⁷, -NR⁶SO₂R⁷ oder zwei R⁵ miteinander verbunden eine Gruppe ausgewählt aus -C₂₋₆-alkylen, -C₂₋₆-alkenylen und -O-C₁₋₆-alkylen-O-;
R⁶ und R⁷ Wasserstoff, -C₁₋₆-alkyl, -C₃₋₆-cycloalkyl;
n 1;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

9. Verbindungen der Formel **1** nach einem der Ansprüche 6-8, worin
R¹ Wasserstoff, Phenyl, optional substituiert mit 1, 2 oder 3 gleichen oder unterschiedlichen Resten R⁵;
R² Methyl oder Ethyl;
R³ Methyl;
R⁴ OH;
R⁵ Halogen, -C₁₋₆-alkyl, -C₁₋₆-haloalkyl, -COOR⁶, -CONR⁶R⁷, -OR⁶, -NR⁶R⁷ oder zwei R⁵ miteinander verbunden bedeuten -O-C₁₋₆-alkylen-O-;
R⁶ und R⁷ Wasserstoff, -C₁₋₆-alkyl;
n 1;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

10. Verbindungen der Formel **1** nach einem der Ansprüche 6-9, worin die Gruppen R¹, R², R³, R⁴ und n die in den Ansprüchen 6-9 genannten Bedeutung haben, **dadurch gekennzeichnet, dass** sie in Form der R-Enantiomere der Formel ***R*-1** 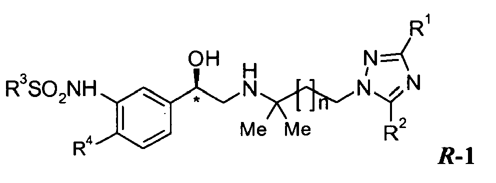 vorliegen.

11. Verwendung der Verbindungen der Formel **1** gemäß einem der Ansprüche 6-10 als Arzneimittel.

12. Verwendung der Verbindungen der Formel **1** gemäß einem der Ansprüche 6-10 zur Herstellung eines Arzneimittels zur Behandlung von Erkrankungen, in denen therapeutisch wirksame Dosen eines Betamimetikum einen therapeutischen Nutzen entfalten können.

13. Pharmazeutische Formulierungen **gekennzeichnet durch** den Gehalt an einer oder mehreren Verbindungen der Formel **1** gemäß einem der Ansprüche 6-10.

14. Inhalativ applizierbare pharmazeutische Formulierung, **gekennzeichnet durch** einen Gehalt einer Verbindung der Formel **1** gemäß einem Ansprüche 1-10.

15. Inhalativ applizierbare pharmazeutische Formulierung nach Anspruch 14, **dadurch gekennzeichnet, dass** sie ausgewählt ist aus der Gruppe bestehend aus Inhalationspulvern, treibgashaltige Dosieraerosole und treibgasfreie Inhalationslösungen.

## Claims

1. Use of compounds of formula **1** 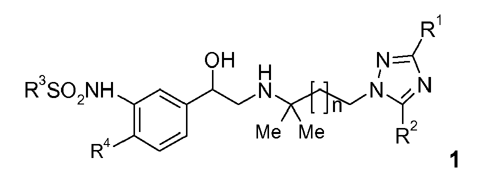 wherein
R¹ denotes hydrogen, -C₁₋₆-alkyl, -C₁₋₆-haloalkyl, -OH, -O-C₁₋₆-alkyl, halogen or a group selected from among aryl or a heterocycle, while aryl or the heterocycle, if possible each optionally substituted by 1, 2, 3, 4 or 5 are identical or different groups R⁵_{;}
R² denotes methyl;
R³ denotes -C₁₋₆-alkyl;
R⁴ denotes -OH;
R⁵ denotes halogen, -CN, -NO₂, -C₁₋₆-alkyl, -C₃₋₆-cycloalkyl, -C₁₋₆-haloalkyl, -COR⁶, -COOR⁶, -CONR⁶R⁷, -OR⁶, -NR⁶R⁷, -NR⁶COR⁷, -NR⁶SO₂R⁷, -SR⁶, -SOR⁶, -SO₂R⁶ or -SO₂NR⁶R⁷, or two R⁵ joined together denote a group selected from -C₂₋₆-alkylene, -C₂₋₆-alkenylene and -O-C₁₋₆-alkylene-O-;
R⁶ and R⁷ denote hydrogen, -C₁₋₆-alkyl, -C₃₋₆-cycloalkyl;
n denotes 0, 1, 2 or 3;
optionally in the form of the individual optical isomers, mixtures of the individual enantiomers or racemates, in the form of the free bases or the corresponding acid addition salts with pharmacologically acceptable acids for preparing a medicament for the treatment of inflammatory and obstructive respiratory complaints

2. Use of compounds of formula **1** according to claim 1, wherein R², R³, R⁴ and n are as hereinbefore defined and
R¹ denotes hydrogen, -C₁₋₆-alkyl, -C₁₋₆-haloalkyl, -OH, -O-C₁₋₆-alkyl, halogen or aryl, if possible optionally substituted by 1, 2, 3, 4 or 5 identical or different groups R⁵;
R⁵ denotes halogen, -CN, -NO₂, -C₁₋₆-alkyl, -C₃₋₆-cycloalkyl, -C₁₋₆-haloalkyl, -COR⁶, -COOR⁶, -CONR⁶R⁷, -OR⁶, -NR⁶R⁷, -NR⁶COR⁷, -NR⁶SO₂R⁷, -SR⁶, -SOR⁶, -SO₂R⁶ or -SO₂NR⁶R⁷, or two R⁵ joined together denote a group selected from -C₂₋₆-alkylene, -C₂₋₆-alkenylene and -O-C₁₋₆-alkylene-O-;
R⁶ and R⁷ denote hydrogen, -C₁₋₆-alkyl, -C₃₋₆-cycloalkyl;
optionally in the form of the individual optical isomers, mixtures of the individual enantiomers or racemates, in the form of the free bases or the corresponding acid addition salts with pharmacologically acceptable acids.

3. Use of compounds of formula 1 according to one of claims 1 or 2, wherein
R¹ denotes hydrogen, -C₁₋₆-alkyl, -O-C₁₋₆-alkyl, aryl, if possible optionally substituted by 1, 2, 3, 4 or 5 identical or different groups R⁵_{;}
R² denotes methyl;
R³ denotes methyl;
R⁴ denotes -OH;
R⁵ denotes halogen, -CN, -NO₂, -C₁₋₆-alkyl, -C₃₋₆-cycloalkyl, -C₁₋₆-haloalkyl, -COR⁶, -COOR⁶, -CONR⁶R⁷, -OR⁶, -NR⁶R⁷, -NR⁶COR⁷, -NR⁶SO₂R⁷, -SR⁶, -SOR⁶, -SO₂R⁶ or -SO₂NR⁶R⁷, or two R⁵ joined together represent a group selected from -C₂₋₆-alkylene, -C₂₋₆-alkenylene and -O-C₁₋₆-alkylene-O-;
R⁶ and R⁷ denote hydrogen, -C₁₋₆-alkyl, -C₃₋₆-cycloalkyl;
n denotes 0, 1, 2 or 3;
optionally in the form of the individual optical isomers, mixtures of the individual enantiomers or racemates, in the form of the free bases or the corresponding acid addition salts with pharmacologically acceptable acids.

4. Use of compounds of formula **1** according to one of claims 1-3, wherein
R¹ denotes hydrogen, aryl, if possible optionally substituted by 1, 2, 3, 4 or 5 identical or different groups R⁵;
R² denotes methyl;
R³ denotes methyl;
R⁴ denotes -OH;
R⁵ denotes halogen, -CN, -NO₂, -C₁₋₆-alkyl, -C₃₋₆-cycloalkyl, -C₁₋₆-haloalkyl, -COR⁶, -COOR⁶, -CONR⁶R⁷, -OR⁶, -NR⁶R⁷, -NR⁶COR⁷, -NR⁶SO₂R⁷, -SR⁶, -SOR⁶, -SO₂R⁶ or -SO₂NR⁶R⁷, or two R⁵ joined together represent a group selected from -C₂₋₆-alkylene, -C₂₋₆-alkenylene and -O-C₁₋₆-alkylene-O-;
R⁶ and R⁷ denote hydrogen, -C₁₋₆-alkyl, -C₃₋₆-cycloalkyl;
n denotes 0, 1, 2 or 3;
optionally in the form of the individual optical isomers, mixtures of the individual enantiomers or racemates, in the form of the free bases or the corresponding acid addition salts with pharmacologically acceptable acids.

5. Use according to one of claims 1 to 5, **characterised in that** the compounds of formula **1** are used in the form of their R-enantiomers.

6. Compounds of formula 1 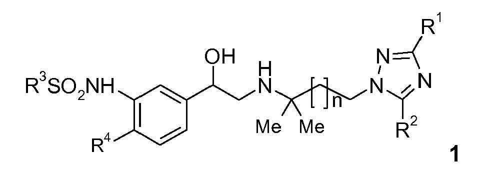 wherein R¹ and n may have the meanings given in claims 1 to 4 and wherein
R² denotes methyl or ethyl;
R³ denotes methyl;
R⁴ denotes OH,
optionally in the form of the individual optical isomers, mixtures of the individual enantiomers or racemates, in the form of the free bases or the corresponding acid addition salts with pharmacologically acceptable acids.

7. Compounds of formula **1** according to claim 6, wherein n = 1, optionally in the form of the individual optical isomers, mixtures of the individual enantiomers or racemates, in the form of the free bases or the corresponding acid addition salts with pharmacologically acceptable acids.

8. Compounds of formula **1** according to one of claims 6 or 7, wherein
R¹ denotes hydrogen, phenyl, optionally substituted by 1, 2 or 3 identical or different groups R⁵;
R² denotes methyl or ethyl;
R³ denotes methyl;
R⁴ denotes OH;
R⁵ denotes halogen, -C₁₋₆-alkyl, -C₃₋₆-cycloalkyl, -C₁₋₆-haloalkyl, -COOR⁶, -CONR⁶R⁷, -OR⁶, -NR⁶R⁷, -NR⁶COR⁷, -NR⁶SO₂R⁷ or two R⁵ joined together denote a group selected from -C₂₋₆-alkylene, -C₂₋₆-alkenylene and -O-C₁₋₆-alkylene-O-;
R⁶ and R⁷ denote hydrogen, -C₁₋₆-alkyl, -C₃₋₆-cycloalkyl;
n denotes 1;
optionally in the form of the individual optical isomers, mixtures of the individual enantiomers or racemates, in the form of the free bases or the corresponding acid addition salts with pharmacologically acceptable acids.

9. Compounds of formula **1** according to one of claims 6-8, wherein
R¹ denotes hydrogen, phenyl, optionally substituted by 1, 2 or 3 identical or different groups R⁵;
R² denotes methyl or ethyl;
R³ denotes methyl;
R⁴ denotes OH;
R⁵ denotes halogen, -C₁₋₆-alkyl, -C₁₋₆-haloalkyl, -COOR⁶, -CONR⁶R⁷, -OR⁶, -NR⁶R⁷ or two R⁵ joined together represent -O-C₁₋₆-alkylene-O-;
R⁶ and R⁷ denote hydrogen, -C₁₋₆-alkyl;
n denotes 1;
optionally in the form of the individual optical isomers, mixtures of the individual enantiomers or racemates, in the form of the free bases or the corresponding acid addition salts with pharmacologically acceptable acids.

10. Compounds of formula **1** according to one of claims 6-9, wherein the groups R¹, R², R³, R⁴ and n are defined as in claims 6-9, **characterised in that** they are in the form of the R-enantiomers of formula ***R*-1**

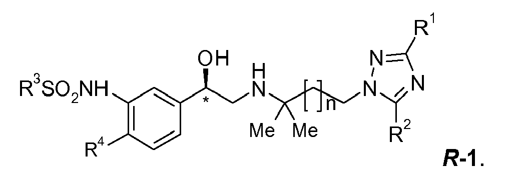

11. Use of the compounds of formula **1** according to one of claims 6 to 10 as medicaments.

12. Use of the compounds of formula **1** according to one of claims 6 to 10 for preparing a medicament for the treatment of diseases in which therapeutically effective doses of a betamimetic may develop a therapeutic benefit.

13. Pharmaceutical formulations, **characterised in that** they contain one or more compounds of formula **1** according to one of claims 6 to 10.

14. Pharmaceutical formulation capable of being administered by inhalation, **characterised in that** it contains a compound of formula 1 according to one of claims 1 to 10.

15. Pharmaceutical formulation capable of being administered by inhalation according to claim 14, **characterised in that** it is selected from among the inhalable powders, propellant-containing metered-dose aerosols and propellant-free inhalable solutions.

## Revendications

1. Utilisation de composés de formule 1 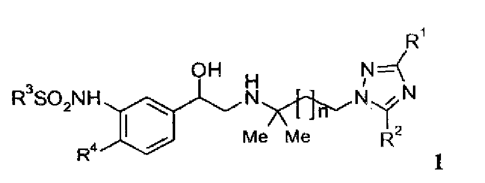 où
R¹ représente l'hydrogène, -C₁₋₆-alkyle, -C₁₋₆-haloalkyle, -OH, -O-C₁₋₆-alkyle, halogène ou un groupement choisi dans le groupe aryle ou hétérocycle, où aryle ou hétérocycle, quand cela est possible, est éventuellement substitué dans chaque cas avec 1, 2, 3, 4 ou 5 groupements R⁵ identiques ou différents ;
R² représente méthyle ;
R³ représente -C₁₋₆-alkyle ;
R⁴ représente -OH ;
R⁵ représente halogène, -CN, -NO₂, -C₁₋₆-alkyle, -C₃₋₆-cycloalkyle, -C₁₋₆-haloalkyle, -COR⁶, -COOR⁶, -CONR⁶R⁷, -OR⁶, -NR⁶R⁷, -NR⁶COR⁷, -NR⁶SO₂R⁷, -SR⁶, -SOR⁶, -SO₂R⁶ ou -SO₂NR⁶R⁷, ou deux R⁵ liés entre eux représentent un groupe choisi parmi -C₂₋₆-alkylène, -C₂₋₆-alcénylène et -O-C₁₋₆-alkylène-O- ;
R⁶ et R⁷ représentent l'hydrogène, -C₁₋₆-alkyle, -C₃₋₆-cycloalkyle ;
n représente 0, 1, 2 ou 3 ;
éventuellement sous forme des isomères optiques individuels, de mélanges des énantiomères individuels ou de racémates, sous forme des bases libres ou des sels d'addition d'acide correspondants avec des acides pharmacologiquement acceptables pour la production d'un médicament pour le traitement des maladies inflammatoires et obstructives des voies respiratoires.

2. Utilisation de composés de formule **1** selon la revendication 1 où R², R³, R⁴ et n ont la signification citée ci-dessus et
R¹ représente l'hydrogène, -C₁₋₆-alkyle, -C₁₋₆-haloalkyle, -OH, -O-C₁-₆-alkyle, halogène ou aryle, quand cela est possible éventuellement substitué avec 1, 2, 3, 4 ou 5 groupements R⁵ identiques ou différents ;
R⁵ représente halogène, -CN, -NO₂, -C₁₋₆-alkyle, -C₃₋₆-cycloalkyle, -C₁₋₆-haloalkyle, -COR⁶, -COOR⁶, -CONR⁶R⁷, -OR⁶, -NR⁶R⁷, -NR⁶COR⁷, -NR⁶SO₂R⁷, -SR⁶, -SOR⁶, -SO₂R⁶ ou -SO₂NR⁶R⁷, ou deux R⁵ liés entre eux représentent un groupe choisi parmi -C₂₋₆-alkylène, -C₂₋₆-alcénylène et -O-C₁₋₆-alkylène-O- ;
R⁶ et R⁷ représentent l'hydrogène, -C₁₋₆-alkyle, -C₃₋₆-cycloalkyle ;
éventuellement sous forme des isomères optiques individuels, de mélanges des énantiomères individuels ou de racémates, sous forme des bases libres ou des sels d'addition d'acide correspondants avec des acides pharmacologiquement acceptables.

3. Utilisation de composés de formule **1** selon l'une des revendications 1 et 2
où
R¹ représente l'hydrogène, -C₁₋₆-alkyle, -O-C₁₋₆-alkyle, aryle, quand cela est possible éventuellement substitué avec 1, 2, 3, 4 ou 5 groupements R⁵ identiques ou différents ;
R² représente méthyle ;
R³ représente méthyle ;
R⁴ représente -OH ;
R⁵ représente halogène, -CN, -NO₂, -C₁₋₆-alkyle, -C₃₋₆-cycloalkyle, -C₁₋₆-haloalkyle, -COR⁶, -COOR⁶, -CONR⁶R⁷, -OR⁶, -NR⁶R⁷, -NR⁶COR⁷, -NR⁶SO₂R⁷, -SR⁶, -SOR⁶, -SO₂R⁶ ou -SO₂NR⁶R⁷, ou deux R⁵ liés entre eux représentent un groupe choisi parmi -C₂₋₆-alkylène, -C₂₋₆-alcénylène et -O-C₁₋₆-alkylène-O- ;
R⁶ et R⁷ représentent l'hydrogène, -C₁₋₆-alkyle, -C₃₋₆-cycloalkyle ;
n représente 0, 1, 2 ou 3 ;
éventuellement sous forme des isomères optiques individuels, de mélanges des énantiomères individuels ou de racémates, sous forme des bases libres ou des sels d'addition d'acide correspondants avec des acides pharmacologiquement acceptables.

4. Utilisation de composés de formule **1** selon l'une des revendications 1-3
où
R¹ représente l'hydrogène, aryle, quand cela est possible éventuellement substitué avec 1, 2, 3, 4 ou 5 groupements R⁵ identiques ou différents ;
R² représente méthyle ;
R³ représente méthyle ;
R⁴ représente -OH ;
R⁵ représente halogène, -CN, -NO₂, -C₁₋₆-alkyle, -C₃₋₆-cycloalkyle, -C₁₋₆-haloalkyle, -COR⁶, -COOR⁶, -CONR⁶R⁷, -OR⁶, -NR⁶R⁷, -NR⁶COR⁷, -NR⁶SO₂R⁷, -SR⁶, -SOR⁶, -SO₂R⁶ ou -SO₂NR⁶R⁷, ou deux R⁵ liés entre eux représentent un groupe choisi parmi -C₂₋₆-alkylène, -C₂₋₆-alcénylène et -O-C₁₋₆-alkylène-O- ;
R⁶ et R⁷ représentent l'hydrogène, -C₁₋₆-alkyle, -C₃₋₆-cycloalkyle ;
n représente 0, 1, 2 ou 3 ;
éventuellement sous forme des isomères optiques individuels, de mélanges des énantiomères individuels ou de racémates, sous forme des bases libres ou des sels d'addition d'acide correspondants avec des acides pharmacologiquement acceptables.

5. Utilisation selon l'une des revendications 1-4 **caractérisée en ce que** les composés de formule **1** sont utilisés sous forme de leurs énantiomères *R*.

6. Composés de formule **1** 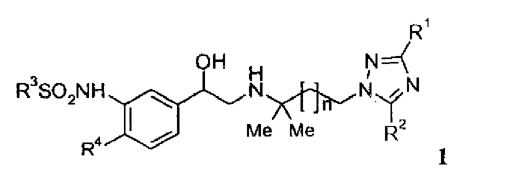
où R¹ et n peuvent avoir les significations citées dans les revendications 1-4 et où
R² représente méthyle ou éthyle ;
R³ représente méthyle ;
R⁴ représente OH ;
éventuellement sous forme des isomères optiques individuels, de mélanges des énantiomères individuels ou de racémates, sous forme des bases libres ou des sels d'addition d'acide correspondants avec des acides pharmacologiquement acceptables.

7. Composés de formule **1** selon la revendication 6 où n = 1, éventuellement sous forme des isomères optiques individuels, de mélanges des énantiomères individuels ou de racémates, sous forme des bases libres ou des sels d'addition d'acide correspondants avec des acides pharmacologiquement acceptables.

8. Composés de formule **1** selon l'une des revendications 6 et 7 où
R¹ représente l'hydrogène, phényle, éventuellement substitué avec 1, 2 ou 3 groupements R⁵ identiques ou différents ;
R² représente méthyle ou éthyle ;
R³ représente méthyle ;
R⁴ représente OH ;
R⁵ représente halogène, -C₁₋₆-alkyle, -C₃₋₆-cycloalkyle, -C₁₋₆-haloalkyle, -COOR⁶, - CONR⁶R⁷, -OR⁶, -NR⁶R⁷, -NR⁶COR⁷, -NR⁶SO₂R⁷ ou deux R⁵ liés entre eux représentent un groupe choisi parmi -C₂₋₆-alkylène, -C₂₋₆-alcénylène et -O-C₁₋₆-alkylène-O- ;
R⁶ et R⁷ représentent l'hydrogène, -C₁₋₆-alkyle, -C₃₋₆-cycloalkyle ;
n représente 1 ;
éventuellement sous forme des isomères optiques individuels, de mélanges des énantiomères individuels ou de racémates, sous forme des bases libres ou des sels d'addition d'acide correspondants avec des acides pharmacologiquement acceptables.

9. Composés de formule **1** selon l'une des revendications 6 - 8 où
R¹ représente l'hydrogène, phényle, éventuellement substitué avec 1, 2 ou 3 groupements R⁵ identiques ou différents ;
R² représente méthyle ou éthyle ;
R³ représente méthyle ;
R⁴ représente OH ;
R⁵ représente halogène, -C₁₋₆-alkyle, -C₁₋₆-haloalkyle, -COOR⁶, -CONR⁶R⁷, -OR⁶, -NR⁶R⁷ ou deux R⁵ liés entre eux représentent -O-C₁₋₆-alkylène-O- ;
R⁶ et R⁷ représentent l'hydrogène, -C₁₋₆-alkyle ;
n représente 1 ;
éventuellement sous forme des isomères optiques individuels, de mélanges des énantiomères individuels ou de racémates, sous forme des bases libres ou des sels d'addition d'acide correspondants avec des acides pharmacologiquement acceptables.

10. Composés de formule **1** selon l'une des revendications 6 - 9 où les groupes R¹, R², R³, R⁴ et n ont la signification citée dans les revendications 6 - 9, **caractérisés en ce qu'**ils sont sous forme des énantiomères R de formule ***R*-1**

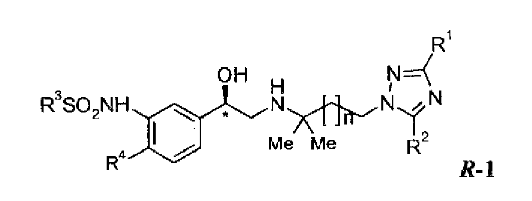

11. Utilisation des composés de formule **1** selon l'une des revendications 6 - 10 comme médicament.

12. Utilisation des composés de formule **1** selon l'une des revendications 6 - 10 pour la production d'un médicament pour le traitement des maladies dans lesquelles des doses thérapeutiquement efficaces d'un bêta-mimétique peuvent présenter une utilité thérapeutique.

13. Formulations pharmaceutiques **caractérisées par** la teneur en un ou plusieurs composés de formule **1** selon l'une des revendications 6 - 10.

14. Formulation pharmaceutique applicable par inhalation **caractérisée par** une teneur d'un composé de formule **1** selon l'une des revendications 1 - 10.

15. Formulation pharmaceutique applicable par inhalation selon la revendication 14 **caractérisée en ce qu'**elle est choisie dans le groupe consistant en les poudres pour inhalation, les aérosols doseurs contenant un gaz propulseur et les solutions pour inhalation sans gaz propulseur.
